# EUROPEAN PATENT APPLICATION

(11) **EP 3 719 133 A1**
(43) Date of publication of application: **07.10.2020**
(21) Application number: 18883491.5
(22) Date of filing: 27.11.2018
(51) Int. Cl.: C12N 15/83

(54) **METHOD FOR PRODUCING NEURAL CELLS**

(30) Priority: 30.11.2017 US 201762592629 P
(71) Applicant: I Peace, Inc., Palo Alto, CA 94303 (US); ID Pharma Co., Ltd., Tokyo 102-0071 (JP)
(72) Inventor: TANABE, Koji, Palo Alto California 94303 (US); INOUE, Makoto, Tokyo 102-0071 (JP); SHU, Tsugumine, Tokyo 102-0071 (JP); MORI, Toyotaka, Tokyo 102-0071 (JP)
(74) Representative: Zacco GmbH
(86) International application number: PCT/JP2018/043573
(87) International publication number: WO 2019/107354

(57) **Abstract**

Provided is a nerve cell production method including preparing stem cells, and introducing Sendai virus by infection to the stem cells to induce the stem cells into nerve cells by allowing the Sendai virus to express mRNA which synthesizes an inducing factor in the stem cells.

SEQUENCE LISTING
<110> I Peace, Inc.

<120> Method for Manufacturing Neuronal Cells

<130> XT374AJP0007-WO

<150> US 62/592,629
<151> 2017-11-30

<160> 10

<170> PatentIn version 3.5.1

<210> 1
<211> 2244
<212> DNA
<213> Artificial Sequence

<220>
<223> Inducing factor

<400> 1

gcagccactg aaccacaagc agctcggctt taactggagt gccttggagt cgcgtgccag 60
cagccacacg gccagggact gactgacaga caaccacgca cgagaacgac aacacacgag 120
actcgggcga gctgccgcgg tcgtccgggc tcttggcaaa gtcgcccagc cgagaggccc 180
ccccgcggag gtgcgcctag gaagcgccaa gcccgcggcg cggaggacac cgtgctcggt 240
tccgggctgc ggggacattc ccggacacac accggagcag cagctgcgcc gcgacacatc 300
tggagccgcg taggatgttc gtcaaatctg agactctgga gttgaaggag gaagaggagg 360
tactgatgct gctgggctcg gcttccccgg cctcggcgac cctgaccccg atgtcctcca 420
gcgcggacga ggaggaggac gaggagctgc gccggccggg ctccgcgcgt gggcagcgtg 480
gagcggaagc cgggcagggg gtgcagggca gtccggcgtc gggtgccggg ggttgccggc 540
cagggcggct gctgggcctg atgcacgagt gcaagcgtcg cccgtcgcgc tcacgggccg 600
tctcccgagg tgccaagacg gcggagacgg tgcagcgcat caagaagacc cgcaggctca 660
aggccaacaa ccgcgagcgc aaccgcatgc acaacctaaa cgccgcgctg gacgcgctgc 720
gcgaggtgct gcccaccttc cccgaggatg ccaagctcac gaagatcgag acgctgcgct 780
tcgcccacaa ttacatctgg gcgctcaccg agactctgcg cctggcggac cactgcgccg 840
gcgccggtgg cctccagggg gcgctcttca cggaggcggt gctcctgagc ccgggagctg 900
cgctcggcgc cagcggggac agcccttctc caccttcctc ctggagctgc accaacagcc 960
cggcgtcatc ctccaactcc acgtccccat acagctgcac tttatcgccc gctagccccg 1020
ggtcagacgt ggactactgg cagcccccac ctccggagaa gcatcgttat gcgcctcacc 1080
tgcccctcgc cagggactgt atctagagct gcgggtctcc ctctctcgtc ctctacccgg 1140
ccctcttccc atccttctcc cgcccctcac cctccacgcc ccggactcca cttcacagag 1200
cagaggtggc ccttgcaatc ccctcggcgg ctggtgcatt cgggggtgga gaccagctct 1260
ggtttattga agatgtgagg atttatggtc aaagaggact atggcgtgtg ggagtggggg 1320
ctggcgtggg gaacctcgta agactgtaaa agacactgag aaaaagtacc ataactaacg 1380
agtgtgcaga gcagactgac gctcctcccc tctctcagag ctgctggagg agaactccgg 1440
gcaggcagtt cgtgtgaatc tctcagaggg aatgcaactg gtccctgtga tcttttcacc 1500
ttcgtttcta catagagatg ttaatgtcag tcgaaagaaa tgtattttag catctgaatg 1560
aatttactgg taataatatt atccacacat ttgcaatggc tggcatctgc tctattccca 1620
ttgctgtctg caggctgtgg gaatttcacc tgtcaaacca aactttccct ctctgatgtg 1680
cactttgttt ttttcccaga ttcgtcacaa tgcctattgt cccgcccttc tttttgcttt 1740
ttttctccat tttgccatct gtctcttatg atttataagg gggaaaaact tgttttgtta 1800
gagggccagg ttagaagtca ttgtataatt tgtaggcttt tgtaagggtt gaatgcaagc 1860
gtggaaattt aggctgaatt ctctatcaaa agaaaaaatg tgaaggaaaa aggaaaaatc 1920
aggagggagg attgcttcat gcattattta tctcgacctt ttaggggaga aggaactccc 1980
ccatcctttc aagagattaa aaataaatca acagtctgaa aacctaagca gacacggggc 2040
attgccagga tcagccacac acgtgtttcc ttctatttat tttgaagaaa aatttcatgg 2100
gaaagtatgt atttttttgt atattctaca gagtttattc tagtatgtat ttacatcccg 2160
aagaataaga aaattgtttt gtgattaagc tataaataaa gtatctaatt ttcataaaaa 2220
aaaaaaaaaa aaaaaaaaaa aaaa 2244

<210> 2
<211> 2370
<212> DNA
<213> Artificial Sequence

<220>
<223> Inducing factor

<400> 2

cgcagccact gaaccacaag cagcttcgcg ttaactggag tgcctgggag tcgcgtgcca 60
ggagccgcac ggccagggac tgactgacag acagacacgc accaccacca caacacacga 120
gacccgggcg ggccgccgcc gccgccgccg gggctcttgg caaactcgcc ggtcgcagag 180
gtcccccgcg gagctgcgcc acagtagcgc cgggcttgca gctttcacgc cgggcgaagg 240
acccggcgct gcgctcgcag ctgcgcggag attcccggca caggccaaag tcacagcaac 300
gctgaggcac agttagagcc aactaagatg ttcgtcaaat ccgagacctt ggagttgaag 360
gaggaagagg acgtgttagt gctgctcgga tcggcctccc ccgccttggc ggccctgacc 420
ccgctgtcat ccagcgccga cgaagaagag gaggaggagc cgggcgcgtc aggcggggcg 480
cgtcggcagc gcggggctga ggccgggcag ggggcgcggg gcggcgtggc tgcgggtgcg 540
gagggctgcc ggcccgcacg gctgctgggt ctggtacacg attgcaaacg gcgcccttcc 600
cgggcgcggg ccgtctcccg aggcgccaag acggccgaga cggtgcagcg catcaagaag 660
acccgtagac tgaaggccaa caaccgcgag cgaaaccgca tgcacaacct caacgcggca 720
ctggacgcgc tgcgcgaggt gctccccacg ttccccgagg acgccaagct caccaagatc 780
gagaccctgc gcttcgccca caactacatc tgggcactca ccgagaccct gcgcctggcg 840
gatcactgcg gggggcggcg cgggggcctg ccgggggcgc tcttctccga ggcagtgttg 900
ctgagcccgg gaggagccag cgccgccctg agcagcagcg gagacagccc ctcgcccgcc 960
tccacgtgga gttgcaccaa cagccccgcg ccgtcctcct ccgtgtcctc caattccacc 1020
tccccctaca gctgcacttt atcgcccgcc agcccggccg ggtcagacat ggactattgg 1080
cagcccccac ctcccgacaa gcaccgctat gcacctcacc tccccatagc cagggattgt 1140
atctagagct gccatttctg ctacccacgc caggccttag tgggttccct ttcctgtccc 1200
cagtcgagcc ctcctccctt cccctgcccc tcctttccac gccctggaaa ccatctcact 1260
tcacagggca ggtgtagcct ttctgattcc tcggttgttt cttgcatttc ttggctttgg 1320
gtatccttca ttcagacggg ctctgattta ctgaaggtgt gatggagctt attgtcaaag 1380
ccaagggtgg cgttttgggg gcgcttcttg agacgaaaaa gaccctggga agagatgatg 1440
gtggcatatc taaagagttt gcagagcgga ctgacgctcc tcccctttct ctttaacgcc 1500
gaaggacttg gtgcagttcg tgtgaatctc acagggggaa tgcaactggt tcctgtgatc 1560
tcttcacctt tgcttctaca tagagatgtt aatgtcgagt agaaagaaat gtatcttagc 1620
atctgaatga ttttgctggt aataatatta tccacagatt tgcaatggct ggcatctgct 1680
ttattcccat tgctgtctgc aggctgtggg aatttcacct gtcaaaccaa acttccctct 1740
ctgatgtgca ctttgttctg tttcccagat tcgtcacaat gcctattgtc ctgtccttct 1800
ctttcctttt tcttccccat tttgccatct gtctcttatg atttataagg ggaaaaaaac 1860
ttgttttgtt agaggggcag gttagaagtc attgtataat ttgtaggctt tgtaatgatt 1920
gaatgcaagc gtggaaattt aggctgaact ctctatcaaa aggaaaaatg tggaggaaaa 1980
gggaaaaatc aggagggagg attgcctcat gtattattta tttcgacctt ttaggggaga 2040
aggaactccc ccattctttc aagagattaa aaataaatca acagtctgaa aacctaagca 2100
gacacggagc attatccgga tcagccacac acgtgttccc ttctatttat tataaagaaa 2160
tttttcatgg gaaaatatgt attttttgta tattctacag agtttattct agtatgtatt 2220
tacatcttga agaacaagaa agttgttctt gtgattaaac tataaataaa ctatctaatt 2280
ttcataaaaa aaaaaaaaaa aaaaaaaaaa aaaaaaaaaa aaaaaaaaaa aaaaaaaaaa 2340
aaaaaaaaaa aaaaaaaaaa aaaaaaaaaa 2370

<210> 3
<211> 2259
<212> DNA
<213> Artificial Sequence

<220>
<223> Inducing factor

<400> 3

agcagtctct cacttctggc cagggaacgt ggaaggcgta ccggctggga gccggttagg 60
gagggcgaat tggggggaac gagagagcaa ttagaaagaa agggggttca accaaataat 120

cccagaagca ggctcaagcc caggctggag caagggagag cgggcgcaag aaagcgcagc 180
cccggagcag ctccacctgg cagagtgcgc tcggcactga cttttgcggc tgctttcctt 240
ttccctttcc tcttttaaaa ccgagaaggc gccggcggcg gccgcacacg cgagcgccac 300
gcgaggctcc cgaagccaac cgcggcggga ggagggaggg gaggaggcgg cgcagaggga 360
agacgatcgc ccaggcacct tcctccgctg cagcctgaca actctgcctc cttctgcgcg 420
tttcttcccc tttaactttc ctccggggct cgtttctccc ctctcctttt tcttcgtccc 480
cctttgatcg tgcttcgcag ccccgcttcc ttcaagggct ctgcgcaccc tgcgtcccca 540
actcgttctc ccccgcgaca gtttggcccg gcatggagag ctctggcaag atggagagtg 600
gagccggcca gcagccgcag cccccgcagc ccttcctgcc tcccgcagcc tgcttctttg 660
cgaccgcggc ggcggcggca gcggcggcgg ccgcggcagc tcagagcgcg cagcagcaac 720
agccgcaggc gccgccgcag caggcgccgc agctgagccc ggtggccgac agccagccct 780
cagggggcgg tcacaagtca gcggccaagc aggtcaagcg ccagcgctcg tcctctccgg 840
aactgatgcg ctgcaaacgc cggctcaact tcagcggctt cggctacagc ctgccacagc 900
agcagccggc cgccgtggcg cgccgcaacg agcgcgagcg caaccgggtc aagttggtca 960
acctgggttt tgccaccctc cgggagcatg tccccaacgg cgcggccaac aagaagatga 1020
gcaaggtgga gacgctgcgc tcggcggtcg agtacatccg cgcgctgcag cagctgctgg 1080
acgagcacga cgcggtgagc gctgcctttc aggcgggcgt cctgtcgccc accatctccc 1140
ccaactactc caacgacttg aactctatgg cgggttctcc ggtctcgtcc tactcctccg 1200
acgagggatc ctacgaccct cttagcccag aggaacaaga gctgctggac tttaccaact 1260
ggttctgagg acctgccagg ctctcctggg aatggacttt ggaagcagga tggcagcaga 1320
tcctgcatct ttagtgtttc tcgccaacga cgtcaaatgg ggaggcagaa aaacaagggg 1380
aaaaaagaag aagaaatgaa acaaacaaac cagacagcca acctacaggg gcaccttcac 1440
taagatgcaa tgttctcagc aaacaggggt gggctccaac agtgtctctg cattccaaca 1500
tcatttccag acacgagaag agtgactggt gtctgaacct aagcccgaat cacagatggg 1560
ttcctttcct ggagcaagag cgtcacacac acacacacac acacagacag acactatatt 1620
aactcccaac cactaacagg cagggctgga agcgcgcatg tgcaagtgcc ttcacctccc 1680
actctctgtc agagctgtct tagccccctg aaactgggtt gatgtctttc ctcagtcacc 1740
cccattccag cgatctatgg acatttgcct ccattgaagc aacgtcagtt ctcggacagc 1800
ctttccctct cctggtggcc tcctccccaa accccacatc gccctcccac ggtctttgct 1860
tctgttttct tcatagaatg cttccaatct ttgtgaattt ttttattata agaaaaaaat 1920
ctatttgtat ctatcctaac cagtttgggg atatattaag atatttttgt acataagaaa 1980
aagagagaga aaaaatttat agaagttttg tacaaatggt ttaaaaatgt gtatatcttg 2040
atactttaac atgtaatgag attacctctg cgtactttag atatgtagtt catcttacaa 2100
ctgccatccc cacccccatc cccagtgtgg ttttggaaag aactctcctc ataggtgaga 2160
tctaaatgcc accagaatga cttcagcacc aatgtgtctt acttcacaga aacgtggtta 2220
atgtattaat gatgttatta aaaaaaactg ttcaagaag 2259

<210> 4
<211> 2490
<212> DNA
<213> Artificial Sequence

<220>
<223> Inducing factor

<400> 4

agcactctct cacttctggc cagggaacgt ggaaggcgca ccgacaggga tccggccagg 60
gagggcgagt gaaagaagga aatcagaaag gaagggagtt aacaaaataa taaaaacagc 120
ctgagccacg gctggagaga ccgagacccg gcgcaagaga gcgcagcctt agtaggagag 180
gaacgcgaga cgcggcagag cgcgttcagc actgactttt gctgctgctt ctgctttttt 240
ttttcttaga aacaagaagg cgccagcggc agcctcacac gcgagcgcca cgcgaggctc 300
ccgaagccaa cccgcgaagg gaggagggga gggaggagga ggcggcgtgc agggaggaga 360
aaaagcattt tcactttttt tgctcccact ctaagaagtc tcccggggat tttgtatata 420
ttttttaact tccgtcaggg ctcccgcttc atatttcctt ttctttccct ctctgttcct 480
gcacccaagt tctctctgtg tccccctcgc gggccccgca cctcgcgtcc cggatcgctc 540
tgattccgcg actccttggc cgccgctgcg catggaaagc tctgccaaga tggagagcgg 600
cggcgccggc cagcagcccc agccgcagcc ccagcagccc ttcctgccgc ccgcagcctg 660
tttctttgcc acggccgcag ccgcggcggc cgcagccgcc gcagcggcag cgcagagcgc 720
gcagcagcag cagcagcagc agcagcagca gcagcaggcg ccgcagctga gaccggcggc 780
cgacggccag ccctcagggg gcggtcacaa gtcagcgccc aagcaagtca agcgacagcg 840
ctcgtcttcg cccgaactga tgcgctgcaa acgccggctc aacttcagcg gctttggcta 900
cagcctgccg cagcagcagc cggccgccgt ggcgcgccgc aacgagcgcg agcgcaaccg 960

cgtcaagttg gtcaacctgg gctttgccac ccttcgggag cacgtcccca acggcgcggc 1020
caacaagaag atgagtaagg tggagacact gcgctcggcg gtcgagtaca tccgcgcgct 1080
gcagcagctg ctggacgagc atgacgcggt gagcgccgcc ttccaggcag gcgtcctgtc 1140
gcccaccatc tcccccaact actccaacga cttgaactcc atggccggct cgccggtctc 1200
atcctactcg tcggacgagg gctcttacga cccgctcagc cccgaggagc aggagcttct 1260
cgacttcacc aactggttct gaggggctcg gcctggtcag gccctggtgc gaatggactt 1320
tggaagcagg gtgatcgcac aacctgcatc tttagtgctt tcttgtcagt ggcgttggga 1380
gggggagaaa aggaaaagaa aaaaaaaaga agaagaagaa gaaaagagaa gaagaaaaaa 1440
acgaaaacag tcaaccaacc ccatcgccaa ctaagcgagg catgcctgag agacatggct 1500
ttcagaaaac gggaagcgct cagaacagta tctttgcact ccaatcattc acggagatat 1560
gaagagcaac tgggacctga gtcaatgcgc aaaatgcagc ttgtgtgcaa aagcagtggg 1620
ctcctggcag aagggagcag cacacgcgtt atagtaactc ccatcacctc taacacgcac 1680
agctgaaagt tcttgctcgg gtcccttcac ctcctcgccc tttcttaaag tgcagttctt 1740
agccctctag aaacgagttg gtgtctttcg tctcagtagc cccaccccca ataagctgta 1800
gacattggtt tacagtgaaa ctatgctatt ctcagccctt tgaaactctg cttctcctcc 1860
agggcccgat tcccaaaccc catggcttcc ctcacactgt cttttctacc attttcatta 1920
tagaatgctt ccaatctttt gtgaattttt tattataaaa aatctatttg tatctatcct 1980
aaccagttcg ggatatattt aagatatttt tgtacataag agagaaagag agagaaaaat 2040
ttatagaagt tttgtacaaa tggtttaaaa tgtgtatatc ttgatacttt aacatgtaat 2100
gctattacct ctgcatattt tagatgtgta gttcacctta caactgcaat tttccctatg 2160
tggttttgta aagaactctc ctcataggtg agatcaagag gccaccagtt gtacttcagc 2220
accaatgtgt cttactttat agaaatgttg ttaatgtatt aatgatgtta ttaaatactg 2280
ttcaagaaga acaaagttta tgcagctact gtccaaactc aaagtggcag ccagttggtt 2340
ttgataggtt gccttttgga gatttctatt actgcctttt tttttcttac tgttttatta 2400
caaacttaca aaaatatgta taaccctgtt ttatacaaac tagtttcgta ataaaacttt 2460
ttcctttttt taaaatgaaa ataaaaaaaa 2490

<210> 5
<211> 7198
<212> DNA
<213> Artificial Sequence

<220>
<223> Inducing factor

<400> 5
   gtggagctgc gtgcattagg ggctggctgt ggaggtaaag cagatctctt aggcccgcta 60
   gcctctcctg ctgcatgtgt cattcctgcc cgcgcgacat tttcccccta agctactgca 120
   cacaaaacag agcgagaaag cttctccctg cagtcttctt ggaggcctcc tggtttctca 180
   cccattgttg gtgggtgtat ttcaattttt tgattccctg gactgtgggt tatgaaatca 240
   atgctgctga agacaaaagc aaccttccct gcctctcggt gctatgcgtg gtcctcctcg 300
   gcctcccact tgtgggggaa agggttttct ctttctttct gtgtgttttg agccagcaca 360
   gttaccaaaa ttgaacttgc cgtcacttgt gagcggtgtg gtcatggtgt gaggggtccc 420
   acagaggctg cagctgaggt ctgggtgtgt gcaattctca gctgggcttt gccctaccca 480
   ggttgacgac tgaagaatca cagagtgtgg aaaagaacac aaggagaaat tggtgagaac 540
   atctgcctag catctccaag tcctgtggag ggagccagca gtgctggtcc aaagaggacc 600
   cagagagtga actcagagtg accacatctg atagaagagg ggaagatgta gtttctgagt 660
   ccagtccagt gtttgtgtct ctcacattgt caacaaaaga aaggctccag ctgtccccac 720
   agacatatgg atattccagg agccacgtaa agatggagaa atggaggcac agagaaatta 780
   agtgacttgg ccacagtcac aagctgggga ggaccaggga aagcctagag agagctggct 840
   ctgggcctgc atcctgccca cggagtcacc ctgcctccgt cctcaggaga gaaggcttcc 900
   tacaagatgg acgtggactc tgaggagaag cgccatcgca cacggtccaa aggggttcga 960
   gttcctgtgg agccagccat acaagagctg ttcagctgtc ccactccagg ctgcgacggc 1020
   agtggtcacg tcagtggcaa atatgcacga cacagaagtg tatatggttg tcccttggct 1080
   aaaaaaagaa aaacgcaaga taaacagccc caagaacctg ctcccaagcg aaaaccattt 1140
   gcagtaaaag cagatagttc ctcagtagac gaatgttatg agagtgatgg tactgaagac 1200
   atggatgata aggaggaaga tgatgatgag gagttctctg aagacaatga tgagcaaggg 1260
   gatgatgacg acgaagatga ggtggatcgg gaagacgagg aggagatcga ggaggaagat 1320
   gatgaagaag atgatgatga tgaagatggt gacgatgtag aagaggaaga agaggatgat 1380
   gatgaagagg aggaagaaga ggaagaggaa gaagaaaatg aagaccatca aatgagttgt 1440
   actcgaataa tgcaggacac agacaaggat gataacaaca atgatgagta tgataactat 1500
   gatgaactgg tagctaagtc gctattaaat cttggcaaaa ttgctgagga tgcagcatac 1560
   cgagccagga ctgaatcaga gatgaacagc aatacctcca atagtctgga ggacgatagt 1620
   gacaaaaacg aaaacctcgg tcggaaaagc gaactgagtc tagacttaga cagtgatgtt 1680
   gttagagaaa cagtggactc ccttaagctg ttagcacaag gacatggtgt tgtgctatca 1740
   gagaatatca gtgacagaag ttatgctgag gggatgtcac agcaggacag tagaaatatg 1800
   aactatgtca tgctagggaa gcccatgaac aatggactca tggagaagat ggtggaggag 1860
   agtgatgagg aagtgtgtct aagtagtcta gagtgcctga ggaaccagtg ctttgacctg 1920
   gccaggaaac tcagcgagac caacccacag gacaggagtc agccacccaa catgagtgtg 1980
   cgccaacatg tccggcaaga ggacgacttc cctgggagga cgccagacag gagctactcg 2040
   gatatgatga accttatgcg gctggaggag cagctcagtc ccaggtctag aacgttctcc 2100
   agctgtgcca aggaggatgg gtgtcatgag agggatgatg acaccacctc agtgaactca 2160
   gacaggtctg aggaagtgtt tgacatgacc aagggcaacc tgactctgct agagaaagcc 2220
   attgccttgg agacagagag agccaaggcc atgcgggaga agatggccat ggatgctggg 2280
   agaagggata acctgagatc ctatgaggac cagtctccaa gacagctggc tggggaagac 2340
   agaaaatcca aatccagtga cagccatgtc aaaaagccat actatggtaa agatccctca 2400
   agaacagaaa agagagagag caagtgtcca acccccgggt gtgatggaac cggccacgta 2460
   actgggcttt acccgcatca ccgcagtctg tctggatgcc cgcacaaaga tagggtccct 2520
   ccagaaattc ttgccatgca tgaaaatgtt ctcaagtgtc ccactccagg ctgcacaggg 2580
   cgagggcatg tgaatagcaa caggaactcg cacagaagcc tctctggatg ccccattgct 2640
   gctgcagaaa aactggcaaa ggcccaagag aaacaccaga gctgtgatgt gtccaaatcc 2700
   aaccaggcct cagaccgagt cctcaggcca atgtgctttg tcaaacagct tgagattcct 2760
   cagtatggct acagaaacaa tgttcccaca accacaccac gctccaacct ggccaaggag 2820
   cttgagaaat actccaagac ttcgtttgag tacaacagtt acgacaacca tacttatggc 2880
   aaaagagcca tagctcccaa ggtgcaaacc agggacatat cccccaaagg atatgacgat 2940
   gccaagcggt actgcaagaa tgccagcccc agcagcagca ccaccagcag ctatgcacct 3000
   agcagcagca gcaacctcag ctgtggtggt ggcagcagcg ccagtagcac gtgtagcaag 3060
   agcagctttg actacacaca tgacatggag gccgcacaca tggcagccac agccattctc 3120
   aacctgtcca cacgttgtcg tgaaatgcca cagaacctgt ccaccaagcc acaggacctg 3180
   tgtactgccc ggaacccaga catggaggtg gatgagaatg gcaccctgga cctgagcatg 3240
   aacaagcaga ggcctcgaga cagctgctgc ccagtcctga cacccctgga acccatgtct 3300
   ccgcagcagc aggccgtgat gagcagccga tgcttccagc tgagcgaggg ggattgctgg 3360
   gacttgcctg tagactacac caaaatgaag cctcggaggg tagatgagga tgagcccaaa 3420
   gagattaccc cagaagactt ggacccattc caggaggctc tggaagaaag acggtatcca 3480
   ggggaggtga ccatcccaag ccccaaaccc aagtaccctc agtgcaagga aagcaaaaag 3540
   gacttaataa ctctgtctgg ctgccccctg gcggacaaaa gcattcgaag tatgctggcc 3600
   accagttccc aagagctcaa gtgccccacc cctggctgtg acggttctgg acacatcact 3660
   ggcaattacg cttctcatcg aagcctttct gggtgcccga gagcaaagaa gagtggcatc 3720
   cggatagcac agagcaaaga ggacaaggaa gaccaggagc caatcaggtg tccggtacct 3780
   ggctgtgacg gtcagggaca catcactggg aagtatgcat cccaccgcag cgcctccggg 3840
   tgtcccttgg cagccaagag gcagaaagat gggtacctta atggctccca gttctcctgg 3900
   aagtcggtca agacggaggg catgtcctgc cctacccccg ggtgtgatgg gtcaggacac 3960
   gtcagtggca gcttcctcac acaccgcagc ttgtcaggat gtccaagagc cacatcagca 4020
   atgaagaaag caaagctgtc tggagaacag atgttgacta tcaagcagcg agccagcaac 4080
   ggtatagaaa atgatgaaga aatcaagcag ttagatgaag agatcaagga gcttaatgag 4140
   tccaattccc agatggaggc tgacatgatc aaactcagaa ctcagatcac cacaatggag 4200
   agcaacctga agacgattga ggaggagaac aaagtcattg aacagcagaa tgagtcgctc 4260
   ttgcacgagt tggccaacct gagccagtcc ctgatccaca gcctcgccaa catccagctg 4320
   cctcacatgg atccaatcaa tgaacaaaat tttgatgctt acgtgactac tttgacggaa 4380
   atgtatacaa atcaagatcg ttatcagagt ccagaaaata aagccctact ggaaaatata 4440
   aagcaggctg tgagaggaat tcaggtctga acagctgctc tagtggtgac tcatgcttaa 4500
   aaaggatgcc tcttgtttct tgctgctgta acttaccaga aagtgttata tatttatttc 4560
   tgtcggaaca gtgttatgct acaagacttc ataatggttt tgtgtgctct cgagagagta 4620
   cctgcagact agttttggat acattcacat tttgtacgtt ttcatataag ctgacatagt 4680
   gtgatttgcc atgtaatgtt tatagctgct gctgtctgca catttggggg tctctatatt 4740
   tctgaagagg taagctgatg aaaataaata gagtgtaaat tctttttaat gctttagtga 4800
   ttaaatgttt tagtattttg aactgaaatg gacaaaaaaa gaaaaaggaa aaaaaaagca 4860
   ggtttgaacg atcactttgt ggcctcctgg ccacttttag acattatcat tttgcctcag 4920
   gttggaggac tttgtggaat ttaagaaata cattttgtgt gcatattgtt tcatagcaag 4980
   aattggttgc aaaaaaatgc tttatttttg aacaatgctt ggaaatatta tgtgactttt 5040
   ttgtttgttt gttttaggag gatggtgtat ggtgggggca ataaatgagg ttttttgcat 5100
   tccaaggaaa tggcatatgg attaactata agaaatgaaa taagtaattt attgtaagac 5160
   aacatcaagc catggaaact tggcagaaga ttcaaagcag cttaaacagc acttttaatt 5220
   aactcctaaa cattacatgg tgggactgtg gagactccgt taagacagga gcttgtcaga 5280
   ggtggacaac acgaagattt cctttgcatt ttcagtaacc tttggagcac acttctctta 5340
   tttctcctag agccccgtgc ccctctgaag tgttaccaca atctagctta cctagcagcc 5400
   gttgattgtt ttttttccat ctgagcaaac aggtaattta agcatttatc tcccctttca 5460
   ctttcaaaaa gaatcatcat tgattattct tgtttcacat ctgaggatga aggctaacgg 5520
   ctgtgcttgc cctggttact ggatgggatt ctctgctggg cgggtgggga agcagctcta 5580
   cccttcccta cccctccctg ccccaactct gacttcgaat aagccttggt cctatccaga 5640
   atacactgga caccaaggcc aaggacgttc acgttctgcc ctcgctgggt ccagctgact 5700
   ctagcgtctg cacagccttg tgtgactcgt ggtgacctaa cctgagaaag agtgcaatca 5760
   gatgttaaag taactaaata gactgcggca cttttttgct ttaaactaga tcatcttaga 5820
   tttgtcgata ccttcgaaat ttgatggttt catcccaaat gactgcacta tatgtatgca 5880
   tacggccact tttgattgct gcgccccttc tgagtagtct ttgacaatgt gttgtgttcc 5940
   ccgatgtcga cttgatttcc ttttagtagc atctctctct tccatgtctt gatgttatgc 6000
   aggaagtaca aaagtacttt aaaattttgt tatgaaataa aaaaaaaaag gatgggtttt 6060
   gtaaaaataa taaaaaaaat atttttagca gaacaggact tacagggtca ttgtccccac 6120
   aatgtgccag tcggctcttt gcactcgcct tgtcctatat atccgtacgg aggtgtgcaa 6180
   tcctgtgtca gtcgccttgt gacactgaag tggatgagtt atagaggagg ccctcgaggc 6240
   tgacccaata cggttactaa gggagactac agggatctca cgacaaacat tctgatacaa 6300
   tactcaacct cggtatatat atatatatat atatatatat acatatatat atatgtataa 6360
   atataagaat atcccagcgg cactttatac tgttcactgt acaaaagctt acagttttcc 6420
   acaaggactt taataactag ctggggaaaa gattatgtaa ttacttgggg ctctgcagga 6480
   ccttctctgt ccagcgcccc ctttctgttg tgcgattagt tgtagctgcc atgctcagaa 6540
   ttgccttttg agagctgaag caaggtgctt actgtcacct gatgccatac acatggtccc 6600
   aggcccacac ccggggggcc tctgttcata gcggcacatg catttcccca ccgcgtcttg 6660
   tctgcagctt cttggccaat gtagtaatgc ttttagtaga gtaataggta gtatcagttt 6720
   ggattcttat tgttatcacc tatgtacaat ggagaggggt tctaagcaca aatctgctgc 6780
   tcatgtaacg gtggtacata atatcaaatc aaaagttatc tgtgactata tatagggatc 6840
   acaaagtgtc acatgttaga atgctgacct tccacatggg gttattgtga gtcatcagag 6900
   catatttatt ataacttatt gttcatattc atttctaagt taatttaagt aatcatttat 6960
   taagacagaa ttttgtataa actatttatt gtgctctctg tggaactgaa gtttgattta 7020
   tttttgtact acacggcatg ggtttgttga cactttaatt ttgctataaa tgtgtggaat 7080
   cacaagttgc tgtgatactt catttttaaa ttgtgaactt tgtacaaact ttgtcatgct 7140
   gatgtgaaca catcttactc tgaataaaaa ggtgttgcca cgtttgtagc acgaagga 7198

<210> 6
<211> 7104
<212> DNA
<213> Artificial Sequence

<220>
<223> Inducing factor

<400> 6

gtctgctgcc tgctgatgga ggctgcgccg catccaccag gcagggctct gcacgcatgc 60
ccgtcttccc cggcgtttag tggtgtcaac ttattaattt ccatcttcga cttcaggcct 120
gttagtttcc tgtctgaaag cattattact tttgtccatg tggcaaaata attagcatac 180
agatgagaag tggccccgtg tctgcgcgcc gatagagaga agatgtgcgg ttcgaagttg 240
attggatgaa tggagaacaa atggtgggcg ttcaaaagca ttagaagagt tttgtagtcg 300
cggagcaggc tggagaattt tctgggggtc ccacagaggc tgcagctgag gtctgggtgt 360
gtgcaattct cagctgggct ttgccctacc caggttgacg actgaagaat cacagagtgt 420
ggaaaagaac acaaggagaa attggtgaga acatctgcct agcatctcca agtcctgtgg 480
agggagccag cagtgctggt ccaaagagga cccagagagt gaactcagag tgaccacatc 540
tgatagaaga ggggaagatg tagtttctga gtccagtcca gtgtttgtgt ctctcacatt 600
gtcaacaaaa gaaaggctcc agctgtcccc acagacatat ggatattcca ggagccacgt 660
aaagatggag aaatggaggc acagagaaat taagtgactt ggccacagtc acaagctggg 720
gaggaccagg gaaagcctag agagagctgg ctctgggcct gcatcctgcc cacggagtca 780
ccctgcctcc gtcctcagga gagaaggctt cctacaagat ggacgtggac tctgaggaga 840
agcgccatcg cacacggtcc aaaggggttc gagttcctgt ggagccagcc atacaagagc 900
tgttcagctg tcccactcca ggctgcgacg gcagtggtca cgtcagtggc aaatatgcac 960
gacacagaag tgtatatggt tgtcccttgg ctaaaaaaag aaaaacgcaa gataaacagc 1020
cccaagaacc tgctcccaag cgaaaaccat ttgcagtaaa agcagatagt tcctcagtag 1080
acgaatgtta tgagagtgat ggtactgaag acatggatga taaggaggaa gatgatgatg 1140
aggagttctc tgaagacaat gatgagcaag gggatgatga cgacgaagat gaggtggatc 1200
gggaagacga ggaggagatc gaggaggaag atgatgaaga agatgatgat gatgaagatg 1260

gtgacgatgt agaagaggaa gaagaggatg atgatgaaga ggaggaagaa gaggaagagg 1320
aagaagaaaa tgaagaccat caaatgagtt gtactcgaat aatgcaggac acagacaagg 1380
atgataacaa caatgatgag tatgataact atgatgaact ggtagctaag tcgctattaa 1440
atcttggcaa aattgctgag gatgcagcat accgagccag gactgaatca gagatgaaca 1500
gcaatacctc caatagtctg gaggacgata gtgacaaaaa cgaaaacctc ggtcggaaaa 1560
gcgaactgag tctagactta gacagtgatg ttgttagaga aacagtggac tcccttaagc 1620
tgttagcaca aggacatggt gttgtgctat cagagaatat cagtgacaga agttatgctg 1680
aggggatgtc acagcaggac agtagaaata tgaactatgt catgctaggg aagcccatga 1740
acaatggact catggagaag atggtggagg agagtgatga ggaagtgtgt ctaagtagtc 1800
tagagtgcct gaggaaccag tgctttgacc tggccaggaa actcagcgag accaacccac 1860
aggacaggag tcagccaccc aacatgagtg tgcgccaaca tgtccggcaa gaggacgact 1920
tccctgggag gacgccagac aggagctact cggatatgat gaaccttatg cggctggagg 1980
agcagctcag tcccaggtct agaacgttct ccagctgtgc caaggaggat gggtgtcatg 2040
agagggatga tgacaccacc tcagtgaact cagacaggtc tgaggaagtg tttgacatga 2100
ccaagggcaa cctgactctg ctagagaaag ccattgcctt ggagacagag agagccaagg 2160
ccatgcggga gaagatggcc atggatgctg ggagaaggga taacctgaga tcctatgagg 2220
accagtctcc aagacagctg gctggggaag acagaaaatc caaatccagt gacagccatg 2280
tcaaaaagcc atactatgat ccctcaagaa cagaaaagag agagagcaag tgtccaaccc 2340
ccgggtgtga tggaaccggc cacgtaactg ggctttaccc gcatcaccgc agtctgtctg 2400
gatgcccgca caaagatagg gtccctccag aaattcttgc catgcatgaa aatgttctca 2460
agtgtcccac tccaggctgc acagggcgag ggcatgtgaa tagcaacagg aactcgcaca 2520
gaagcctctc tggatgcccc attgctgctg cagaaaaact ggcaaaggcc caagagaaac 2580
accagagctg tgatgtgtcc aaatccaacc aggcctcaga ccgagtcctc aggccaatgt 2640
gctttgtcaa acagcttgag attcctcagt atggctacag aaacaatgtt cccacaacca 2700
caccacgctc caacctggcc aaggagcttg agaaatactc caagacttcg tttgagtaca 2760
acagttacga caaccatact tatggcaaaa gagccatagc tcccaaggtg caaaccaggg 2820
acatatcccc caaaggatat gacgatgcca agcggtactg caagaatgcc agccccagca 2880
gcagcaccac cagcagctat gcacctagca gcagcagcaa cctcagctgt ggtggtggca 2940
gcagcgccag tagcacgtgt agcaagagca gctttgacta cacacatgac atggaggccg 3000
cacacatggc agccacagcc attctcaacc tgtccacacg ttgtcgtgaa atgccacaga 3060
acctgtccac caagccacag gacctgtgta ctgcccggaa cccagacatg gaggtggatg 3120
agaatggcac cctggacctg agcatgaaca agcagaggcc tcgagacagc tgctgcccag 3180
tcctgacacc cctggaaccc atgtctccgc agcagcaggc cgtgatgagc agccgatgct 3240
tccagctgag cgagggggat tgctgggact tgcctgtaga ctacaccaaa atgaagcctc 3300
ggagggtaga tgaggatgag cccaaagaga ttaccccaga agacttggac ccattccagg 3360
aggctctgga agaaagacgg tatccagggg aggtgaccat cccaagcccc aaacccaagt 3420
accctcagtg caaggaaagc aaaaaggact taataactct gtctggctgc cccctggcgg 3480
acaaaagcat tcgaagtatg ctggccacca gttcccaaga gctcaagtgc cccacccctg 3540
gctgtgacgg ttctggacac atcactggca attacgcttc tcatcgaagc ctttctgggt 3600
gcccgagagc aaagaagagt ggcatccgga tagcacagag caaagaggac aaggaagacc 3660
aggagccaat caggtgtccg gtacctggct gtgacggtca gggacacatc actgggaagt 3720
atgcatccca ccgcagcgcc tccgggtgtc ccttggcagc caagaggcag aaagatgggt 3780
accttaatgg ctcccagttc tcctggaagt cggtcaagac ggagggcatg tcctgcccta 3840
cccccgggtg tgatgggtca ggacacgtca gtggcagctt cctcacacac cgcagcttgt 3900
caggatgtcc aagagccaca tcagcaatga agaaagcaaa gctgtctgga gaacagatgt 3960
tgactatcaa gcagcgagcc agcaacggta tagaaaatga tgaagaaatc aagcagttag 4020
atgaagagat caaggagctt aatgagtcca attcccagat ggaggctgac atgatcaaac 4080
tcagaactca gatcaccaca atggagagca acctgaagac gattgaggag gagaacaaag 4140
tcattgaaca gcagaatgag tcgctcttgc acgagttggc caacctgagc cagtccctga 4200
tccacagcct cgccaacatc cagctgcctc acatggatcc aatcaatgaa caaaattttg 4260
atgcttacgt gactactttg acggaaatgt atacaaatca agatcgttat cagagtccag 4320
aaaataaagc cctactggaa aatataaagc aggctgtgag aggaattcag gtctgaacag 4380
ctgctctagt ggtgactcat gcttaaaaag gatgcctctt gtttcttgct gctgtaactt 4440
accagaaagt gttatatatt tatttctgtc ggaacagtgt tatgctacaa gacttcataa 4500
tggttttgtg tgctctcgag agagtacctg cagactagtt ttggatacat tcacattttg 4560
tacgttttca tataagctga catagtgtga tttgccatgt aatgtttata gctgctgctg 4620
tctgcacatt tgggggtctc tatatttctg aagaggtaag ctgatgaaaa taaatagagt 4680
gtaaattctt tttaatgctt tagtgattaa atgttttagt attttgaact gaaatggaca 4740
aaaaaagaaa aaggaaaaaa aaagcaggtt tgaacgatca ctttgtggcc tcctggccac 4800
ttttagacat tatcattttg cctcaggttg gaggactttg tggaatttaa gaaatacatt 4860
ttgtgtgcat attgtttcat agcaagaatt ggttgcaaaa aaatgcttta tttttgaaca 4920
atgcttggaa atattatgtg acttttttgt ttgtttgttt taggaggatg gtgtatggtg 4980
ggggcaataa atgaggtttt ttgcattcca aggaaatggc atatggatta actataagaa 5040

atgaaataag taatttattg taagacaaca tcaagccatg gaaacttggc agaagattca 5100
aagcagctta aacagcactt ttaattaact cctaaacatt acatggtggg actgtggaga 5160
ctccgttaag acaggagctt gtcagaggtg gacaacacga agatttcctt tgcattttca 5220
gtaacctttg gagcacactt ctcttatttc tcctagagcc ccgtgcccct ctgaagtgtt 5280
accacaatct agcttaccta gcagccgttg attgtttttt ttccatctga gcaaacaggt 5340
aatttaagca tttatctccc ctttcacttt caaaaagaat catcattgat tattcttgtt 5400
tcacatctga ggatgaaggc taacggctgt gcttgccctg gttactggat gggattctct 5460
gctgggcggg tggggaagca gctctaccct tccctacccc tccctgcccc aactctgact 5520
tcgaataagc cttggtccta tccagaatac actggacacc aaggccaagg acgttcacgt 5580
tctgccctcg ctgggtccag ctgactctag cgtctgcaca gccttgtgtg actcgtggtg 5640
acctaacctg agaaagagtg caatcagatg ttaaagtaac taaatagact gcggcacttt 5700
tttgctttaa actagatcat cttagatttg tcgatacctt cgaaatttga tggtttcatc 5760
ccaaatgact gcactatatg tatgcatacg gccacttttg attgctgcgc cccttctgag 5820
tagtctttga caatgtgttg tgttccccga tgtcgacttg atttcctttt agtagcatct 5880
ctctcttcca tgtcttgatg ttatgcagga agtacaaaag tactttaaaa ttttgttatg 5940
aaataaaaaa aaaaaggatg ggttttgtaa aaataataaa aaaaatattt ttagcagaac 6000
aggacttaca gggtcattgt ccccacaatg tgccagtcgg ctctttgcac tcgccttgtc 6060
ctatatatcc gtacggaggt gtgcaatcct gtgtcagtcg ccttgtgaca ctgaagtgga 6120
tgagttatag aggaggccct cgaggctgac ccaatacggt tactaaggga gactacaggg 6180
atctcacgac aaacattctg atacaatact caacctcggt atatatatat atatatatat 6240
atatatacat atatatatat gtataaatat aagaatatcc cagcggcact ttatactgtt 6300
cactgtacaa aagcttacag ttttccacaa ggactttaat aactagctgg ggaaaagatt 6360
atgtaattac ttggggctct gcaggacctt ctctgtccag cgcccccttt ctgttgtgcg 6420
attagttgta gctgccatgc tcagaattgc cttttgagag ctgaagcaag gtgcttactg 6480
tcacctgatg ccatacacat ggtcccaggc ccacacccgg ggggcctctg ttcatagcgg 6540
cacatgcatt tccccaccgc gtcttgtctg cagcttcttg gccaatgtag taatgctttt 6600
agtagagtaa taggtagtat cagtttggat tcttattgtt atcacctatg tacaatggag 6660
aggggttcta agcacaaatc tgctgctcat gtaacggtgg tacataatat caaatcaaaa 6720
gttatctgtg actatatata gggatcacaa agtgtcacat gttagaatgc tgaccttcca 6780
catggggtta ttgtgagtca tcagagcata tttattataa cttattgttc atattcattt 6840
ctaagttaat ttaagtaatc atttattaag acagaatttt gtataaacta tttattgtgc 6900
tctctgtgga actgaagttt gatttatttt tgtactacac ggcatgggtt tgttgacact 6960
ttaattttgc tataaatgtg tggaatcaca agttgctgtg atacttcatt tttaaattgt 7020
gaactttgta caaactttgt catgctgatg tgaacacatc ttactctgaa taaaaaggtg 7080
ttgccacgtt tgtagcacga agga 7104

<210> 7
<211> 7189
<212> DNA
<213> Artificial Sequence

<220>
<223> Inducing factor

<400> 7

gtggagctgc gtgcattagg ggctggctgt ggaggtaaag cagatctctt aggcccgcta 60
gcctctcctg ctgcatgtgt cattcctgcc cgcgcgacat tttcccccta agctactgca 120
cacaaaacag agcgagaaag cttctccctg cagtcttctt ggaggcctcc tggtttctca 180
cccattgttg gtgggtgtat ttcaattttt tgattccctg gactgtgggt tatgaaatca 240
atgctgctga agacaaaagc aaccttccct gcctctcggt gctatgcgtg gtcctcctcg 300
gcctcccact tgtgggggaa agggttttct ctttctttct gtgtgttttg agccagcaca 360
gttaccaaaa ttgaacttgc cgtcacttgt gagcggtgtg gtcatggtgt gaggggtccc 420
acagaggctg cagctgaggt ctgggtgtgt gcaattctca gctgggcttt gccctaccca 480
ggttgacgac tgaagaatca cagagtgtgg aaaagaacac aaggagaaat tggtgagaac 540
atctgcctag catctccaag tcctgtggag ggagccagca gtgctggtcc aaagaggacc 600
cagagagtga actcagagtg accacatctg atagaagagg ggaagatgta gtttctgagt 660
ccagtccagt gtttgtgtct ctcacattgt caacaaaaga aaggctccag ctgtccccac 720
agacatatgg atattccagg agccacgtaa agatggagaa atggaggcac agagaaatta 780
agtgacttgg ccacagtcac aagctgggga ggaccaggga aagcctagag agagctggct 840
ctgggcctgc atcctgccca cggagtcacc ctgcctccgt cctcaggaga gaaggcttcc 900
tacaagatgg acgtggactc tgaggagaag cgccatcgca cacggtccaa aggggttcga 960
gttcctgtgg agccagccat acaagagctg ttcagctgtc ccactccagg ctgcgacggc 1020

agtggtcacg tcagtggcaa atatgcacga cacagaagtg tatatggttg tcccttggct 1080
aaaaaaagaa aaacgcaaga taaacagccc caagaacctg ctcccaagcg aaaaccattt 1140
gcagtaaaag cagatagttc ctcagtagac gaatgttatg agagtgatgg tactgaagac 1200
atggatgata aggaggaaga tgatgatgag gagttctctg aagacaatga tgagcaaggg 1260
gatgatgacg acgaagatga ggtggatcgg gaagacgagg aggagatcga ggaggaagat 1320
gatgaagaag atgatgatga tgaagatggt gacgatgtag aagaggaaga agaggatgat 1380
gatgaagagg aggaagaaga ggaagaggaa gaagaaaatg accatcaaat gagttgtact 1440
cgaataatgc aggacacaga caaggatgat aacaacaatg atgagtatga taactatgat 1500
gaactggtag ctaagtcgct attaaatctt ggcaaaattg ctgaggatgc agcataccga 1560
gccaggactg aatcagagat gaacagcaat acctccaata gtctggagga cgatagtgac 1620
aaaaacgaaa acctcggtcg gaaaagcgaa ctgagtctag acttagacag tgatgttgtt 1680
agagaaacag tggactccct taagctgtta gcacaaggac atggtgttgt gctatcagag 1740
aatatcagtg acagaagtta tgctgagggg atgtcacagc aggacagtag aaatatgaac 1800
tatgtcatgc tagggaagcc catgaacaat ggactcatgg agaagatggt ggaggagagt 1860
gatgaggaag tgtgtctaag tagtctagag tgcctgagga accagtgctt tgacctggcc 1920
aggaaactca gcgagaccaa cccacaggac aggagtcagc cacccaacat gagtgtgcgc 1980
caacatgtcc ggcaagagga cgacttccct gggaggacgc cagacaggag ctactcggat 2040
atgatgaacc ttatgcggct ggaggagcag ctcagtccca ggtctagaac gttctccagc 2100
tgtgccaagg aggatgggtg tcatgagagg gatgatgaca ccacctcagt gaactcagac 2160
aggtctgagg aagtgtttga catgaccaag ggcaacctga ctctgctaga gaaagccatt 2220
gccttggaga cagagagagc caaggccatg cgggagaaga tggccatgga tgctgggaga 2280
agggataacc tgagatccta tgaggaccag tctccaagac agctggctgg ggaagacaga 2340
aaatccaaat ccagtgacag ccatgtcaaa aagccatact atgatccctc aagaacagaa 2400
aagagagaga gcaagtgtcc aacccccggg tgtgatggaa ccggccacgt aactgggctt 2460
tacccgcatc accgcagtct gtctggatgc ccgcacaaag atagggtccc tccagaaatt 2520
cttgccatgc atgaaaatgt tctcaagtgt cccactccag gctgcacagg gcgagggcat 2580
gtgaatagca acaggaactc gcacagaagc ctctctggat gccccattgc tgctgcagaa 2640
aaactggcaa aggcccaaga gaaacaccag agctgtgatg tgtccaaatc caaccaggcc 2700
tcagaccgag tcctcaggcc aatgtgcttt gtcaaacagc ttgagattcc tcagtatggc 2760
tacagaaaca atgttcccac aaccacacca cgctccaacc tggccaagga gcttgagaaa 2820
tactccaaga cttcgtttga gtacaacagt tacgacaacc atacttatgg caaaagagcc 2880
atagctccca aggtgcaaac cagggacata tcccccaaag gatatgacga tgccaagcgg 2940
tactgcaaga atgccagccc cagcagcagc accaccagca gctatgcacc tagcagcagc 3000
agcaacctca gctgtggtgg tggcagcagc gccagtagca cgtgtagcaa gagcagcttt 3060
gactacacac atgacatgga ggccgcacac atggcagcca cagccattct caacctgtcc 3120
acacgttgtc gtgaaatgcc acagaacctg tccaccaagc cacaggacct gtgtactgcc 3180
cggaacccag acatggaggt ggatgagaat ggcaccctgg acctgagcat gaacaagcag 3240
aggcctcgag acagctgctg cccagtcctg acacccctgg aacccatgtc tccgcagcag 3300
caggccgtga tgagcagccg atgcttccag ctgagcgagg gggattgctg ggacttgcct 3360
gtagactaca ccaaaatgaa gcctcggagg gtagatgagg atgagcccaa agagattacc 3420
ccagaagact tggacccatt ccaggaggct ctggaagaaa gacggtatcc aggggaggtg 3480
accatcccaa gccccaaacc caagtaccct cagtgcaagg aaagcaaaaa ggacttaata 3540
actctgtctg gctgccccct ggcggacaaa agcattcgaa gtatgctggc caccagttcc 3600
caagagctca agtgccccac ccctggctgt gacggttctg gacacatcac tggcaattac 3660
gcttctcatc gaagcctttc tgggtgcccg agagcaaaga agagtggcat ccggatagca 3720
cagagcaaag aggacaagga agaccaggag ccaatcaggt gtccggtacc tggctgtgac 3780
ggtcagggac acatcactgg gaagtatgca tcccaccgca gcgcctccgg gtgtcccttg 3840
gcagccaaga ggcagaaaga tgggtacctt aatggctccc agttctcctg gaagtcggtc 3900
aagacggagg gcatgtcctg ccctaccccc gggtgtgatg ggtcaggaca cgtcagtggc 3960
agcttcctca cacaccgcag cttgtcagga tgtccaagag ccacatcagc aatgaagaaa 4020
gcaaagctgt ctggagaaca gatgttgact atcaagcagc gagccagcaa cggtatagaa 4080
aatgatgaag aaatcaagca gttagatgaa gagatcaagg agcttaatga gtccaattcc 4140
cagatggagg ctgacatgat caaactcaga actcagatca ccacaatgga gagcaacctg 4200
aagacgattg aggaggagaa caaagtcatt gaacagcaga atgagtcgct cttgcacgag 4260
ttggccaacc tgagccagtc cctgatccac agcctcgcca acatccagct gcctcacatg 4320
gatccaatca atgaacaaaa ttttgatgct tacgtgacta ctttgacgga aatgtataca 4380
aatcaagatc gttatcagag tccagaaaat aaagccctac tggaaaatat aaagcaggct 4440
gtgagaggaa ttcaggtctg aacagctgct ctagtggtga ctcatgctta aaaaggatgc 4500
ctcttgtttc ttgctgctgt aacttaccag aaagtgttat atatttattt ctgtcggaac 4560
agtgttatgc tacaagactt cataatggtt ttgtgtgctc tcgagagagt acctgcagac 4620
tagttttgga tacattcaca ttttgtacgt tttcatataa gctgacatag tgtgatttgc 4680
catgtaatgt ttatagctgc tgctgtctgc acatttgggg gtctctatat ttctgaagag 4740
gtaagctgat gaaaataaat agagtgtaaa ttctttttaa tgctttagtg attaaatgtt 4800

ttagtatttt gaactgaaat ggacaaaaaa agaaaaagga aaaaaaaagc aggtttgaac 4860
gatcactttg tggcctcctg gccactttta gacattatca ttttgcctca ggttggagga 4920
ctttgtggaa tttaagaaat acattttgtg tgcatattgt ttcatagcaa gaattggttg 4980
caaaaaaatg ctttattttt gaacaatgct tggaaatatt atgtgacttt tttgtttgtt 5040
tgttttagga ggatggtgta tggtgggggc aataaatgag gttttttgca ttccaaggaa 5100
atggcatatg gattaactat aagaaatgaa ataagtaatt tattgtaaga caacatcaag 5160
ccatggaaac ttggcagaag attcaaagca gcttaaacag cacttttaat taactcctaa 5220
acattacatg gtgggactgt ggagactccg ttaagacagg agcttgtcag aggtggacaa 5280
cacgaagatt tcctttgcat tttcagtaac ctttggagca cacttctctt atttctccta 5340
gagccccgtg cccctctgaa gtgttaccac aatctagctt acctagcagc cgttgattgt 5400
tttttttcca tctgagcaaa caggtaattt aagcatttat ctcccctttc actttcaaaa 5460
agaatcatca ttgattattc ttgtttcaca tctgaggatg aaggctaacg gctgtgcttg 5520
ccctggttac tggatgggat tctctgctgg gcgggtgggg aagcagctct acccttccct 5580
acccctccct gccccaactc tgacttcgaa taagccttgg tcctatccag aatacactgg 5640
acaccaaggc caaggacgtt cacgttctgc cctcgctggg tccagctgac tctagcgtct 5700
gcacagcctt gtgtgactcg tggtgaccta acctgagaaa gagtgcaatc agatgttaaa 5760
gtaactaaat agactgcggc acttttttgc tttaaactag atcatcttag atttgtcgat 5820
accttcgaaa tttgatggtt tcatcccaaa tgactgcact atatgtatgc atacggccac 5880
ttttgattgc tgcgcccctt ctgagtagtc tttgacaatg tgttgtgttc cccgatgtcg 5940
acttgatttc cttttagtag catctctctc ttccatgtct tgatgttatg caggaagtac 6000
aaaagtactt taaaattttg ttatgaaata aaaaaaaaaa ggatgggttt tgtaaaaata 6060
ataaaaaaaa tatttttagc agaacaggac ttacagggtc attgtcccca caatgtgcca 6120
gtcggctctt tgcactcgcc ttgtcctata tatccgtacg gaggtgtgca atcctgtgtc 6180
agtcgccttg tgacactgaa gtggatgagt tatagaggag gccctcgagg ctgacccaat 6240
acggttacta agggagacta cagggatctc acgacaaaca ttctgataca atactcaacc 6300
tcggtatata tatatatata tatatatata tacatatata tatatgtata aatataagaa 6360
tatcccagcg gcactttata ctgttcactg tacaaaagct tacagttttc cacaaggact 6420
ttaataacta gctggggaaa agattatgta attacttggg gctctgcagg accttctctg 6480
tccagcgccc cctttctgtt gtgcgattag ttgtagctgc catgctcaga attgcctttt 6540
gagagctgaa gcaaggtgct tactgtcacc tgatgccata cacatggtcc caggcccaca 6600
cccggggggc ctctgttcat agcggcacat gcatttcccc accgcgtctt gtctgcagct 6660
tcttggccaa tgtagtaatg cttttagtag agtaataggt agtatcagtt tggattctta 6720
ttgttatcac ctatgtacaa tggagagggg ttctaagcac aaatctgctg ctcatgtaac 6780
ggtggtacat aatatcaaat caaaagttat ctgtgactat atatagggat cacaaagtgt 6840
cacatgttag aatgctgacc ttccacatgg ggttattgtg agtcatcaga gcatatttat 6900
tataacttat tgttcatatt catttctaag ttaatttaag taatcattta ttaagacaga 6960
attttgtata aactatttat tgtgctctct gtggaactga agtttgattt atttttgtac 7020
tacacggcat gggtttgttg acactttaat tttgctataa atgtgtggaa tcacaagttg 7080
ctgtgatact tcatttttaa attgtgaact ttgtacaaac tttgtcatgc tgatgtgaac 7140
acatcttact ctgaataaaa aggtgttgcc acgtttgtag cacgaagga 7189

<210> 8
<211> 7152
<212> DNA
<213> Artificial Sequence

<220>
<223> Inducing factor

<400> 8

taagctactg cacactaaac agtgagagag cttttccctg cagtcttgtt gaagcacccc 60
gggttttttg ctcattgttg gtgggtgcat tttaattttt tcattccctg gactatgggt 120
tatgatatcc atactcactg aagacaaaaa gccacctttt ctgcgtcttg gtggcatgca 180
tgtgtctcat catcctttca aacttgtggt ggaacagggt tttcttccct gtctgtgtat 240
tttgagccag cacagttacc aaaattgaac ttgtctttcg cttgtgagcg gttgtggtca 300
ttgtgagggc gggtcatgag gaggctgtag ccaaggacga ggtgtgtgcg gctgttgcct 360
ggacgtttgt ccaatccacg ttgacatttg agggatcaca gcgtgtgaaa atgaactcag 420
aggagaattg gtgaattcct atccagtggg catcttcaaa ccctggtcga cggcggaaga 480
atatcaggtc ctgagatcac ccacccggcg cggcaacagt gcagagtggc cacatctggt 540
ggaagaagaa aaaaatgtag ttattgaatt caatcaagtg tttgcatctt tcaagctatc 600
aacaaaattc catcaagaaa ggttccagtt ggtctcacag acgtatggat atccgaggag 660
ccacctaaag atggagaaat caaggcatag agagattaag tgactttgcc acagtcacaa 720

gctggagagg accaggagta gagcttagag cgagcccctg actctgggcc tgcgtcctgc 780
caggagtcac gctgcctccg ttcctaggag agaagacttc ctgtaagatg gaggtggaca 840
ccgaggagaa gcggcatcgc acgcggtcca aaggggttcg agttcccgtg gaaccagcca 900
tacaagagct gttcagctgt cccacccctg gctgtgacgg cagtggtcat gtcagtggca 960
aatatgcaag acacagaagt gtatatggtt gtcccttggc gaaaaaaaga aaaacacaag 1020
ataaacagcc ccaggaacct gctcctaaac gaaagccatt tgccgtgaaa gcagacagct 1080
cctcagtgga tgagtgtgac gacagtgatg ggactgagga catggatgag aaggaggagg 1140
atgaggggga ggagtactcc gaggacaatg acgagccagg ggatgaggac gaggaggacg 1200
aggaggggga ccgggaggag gaggaggaga tcgaggagga ggatgaggac gatgacgagg 1260
atggagaaga tgtggaggat gaagaagagg aagaggagga ggaggaggag gaggaagagg 1320
aagaagaaaa cgaagaccat caaatgaatt gtcacaatac tcgaataatg caagacacag 1380
aaaaggatga taacaataat gacgaatatg acaattacga tgaactggtg gccaagtcat 1440
tgttaaacct cggcaaaatc gctgaggatg cagcctaccg ggccaggact gagtcagaaa 1500
tgaacagcaa tacctccaat agtctggaag acgatagtga caaaaacgaa aacctgggtc 1560
ggaaaagtga gttgagttta gacttagaca gtgatgttgt tagagaaaca gtggactccc 1620
ttaaactatt agcccaagga cacggtgttg tgctctcaga aaacatgaat gacagaaatt 1680
atgcagacag catgtcgcag caagacagta gaaatatgaa ttacgtcatg ttggggaagc 1740
ccatgaacaa cggactcatg gaaaagatgg tggaggagag cgatgaggag gtgtgtctga 1800
gcagtctgga gtgtttgagg aatcagtgct tcgacctggc caggaagctc agtgagacca 1860
acccgcagga gaggaatccg cagcagaaca tgaacatccg tcagcatgtc cggccagaag 1920
aggacttccc cggaaggacg ccggacagaa actactcgga catgctgaac ctcatgcggc 1980
tggaggagca gttgagcccc cggtcgagag tgtttgccag ctgtgcgaag gaggatgggt 2040
gtcatgagcg ggacgacgat accacctctg tgaactcgga caggtctgaa gaggtgttcg 2100
acatgaccaa ggggaacctg accctgctgg agaaagccat cgctttggaa acggaaagag 2160
caaaggccat gagggagaag atggccatgg aagctgggag gagggacaat atgaggtcat 2220
atgaggacca gtctccgaga caacttcccg gggaggacag aaagcctaaa tccagtgaca 2280
gccatgtcaa aaagccatac tatgatccct caagaacaga aaagaaagag agcaagtgtc 2340
caacccccgg gtgtgatgga accggccacg taactgggct gtacccacat caccgcagcc 2400
tgtccggatg cccgcacaaa gatagggtcc ctccagaaat ccttgccatg catgaaagtg 2460
tcctcaagtg ccccactccg ggctgcacgg ggcgcgggca tgtcaacagc aacaggaact 2520
cccaccgaag cctctccgga tgcccgatcg ctgcagcaga gaaactggcc aaggcacagg 2580
aaaagcacca gagctgcgac gtgtccaagt ccagccaggc ctcggaccgc gtgctcaggc 2640
caatgtgctt tgtgaagcag ctggagattc ctcagtatgg ctacagaaac aatgtcccca 2700
caactacgcc gcgttccaac ctggccaagg agctcgagaa atattccaag acctcgtttg 2760
aatacaacag ttacgacaac catacttatg gcaagcgagc catagctccc aaggtgcaaa 2820
ccagggatat atcccccaaa ggatatgatg atgcgaagcg gtactgcaag gaccccagcc 2880
ccagcagcag cagcaccagc agctacgcgc ccagcagcag cagcaacctg agctgcggcg 2940
ggggcagcag cgccagcagc acgtgcagca agagcagctt cgactacacg cacgacatgg 3000
aggcggccca catggcggcc accgccatcc tcaacctgtc cacgcgctgc cgcgagatgc 3060
cgcagaacct gagcaccaag ccgcaggacc tgtgcgccac gcggaaccct gacatggagg 3120
tggatgagaa cgggaccctg gacctcagca tgaacaagca gaggccgcgg gacagctgct 3180
gccccatcct gacccctctg gagcccatgt ccccccagca gcaggcagtg atgaacaacc 3240
ggtgtttcca gctgggcgag ggcgactgct gggacttgcc cgtagactac accaaaatga 3300
aaccccggag gatagacgag gacgagtcca aagacattac tccagaagac ttggacccat 3360
tccaggaggc tctagaagaa agacggtatc ccggggaggt gaccatccca agtcccaaac 3420
ccaagtaccc tcagtgcaag gagagcaaaa aggacttaat aactctgtct ggctgccccc 3480
tggcggacaa aagcattcga agtatgctgg ccaccagctc ccaagaactc aagtgcccca 3540
cgcctggctg tgatggttct ggacatatca ccggcaatta tgcttctcat cggagccttt 3600
caggttgccc aagagcaaag aaaagtggta tcaggatagc acagagcaaa gaagataaag 3660
aagatcaaga acccatcagg tgtccggtcc ccgggtgcga cggccagggc cacatcactg 3720
ggaagtacgc gtcccatcgc agcgcctccg ggtgcccctt ggcggccaag aggcagaaag 3780
acgggtacct gaatggctcc cagttctcct ggaagtcggt caagacggaa ggcatgtcct 3840
gccccacgcc aggatgcgac ggctcaggcc acgtcagcgg cagcttcctc acacaccgca 3900
gcttgtcagg atgcccgaga gccacgtcag cgatgaagaa ggcaaagctt tctggagagc 3960
agatgctgac catcaaacag cgggccagca acggtataga aaatgatgaa gaaatcaaac 4020
agttagatga agaaatcaag gagctaaatg aatccaattc ccagatggaa gccgatatga 4080
ttaaactcag aactcagatt accacgatgg agagcaacct gaagaccatc gaagaggaga 4140
acaaagtgat tgagcagcag aacgagtctc tcctccacga gctggcgaac ctgagccagt 4200
ctctgatcca cagcctggct aacatccagc tgccgcacat ggatccaatc aatgaacaaa 4260
attttgatgc ttacgtgact actttgacgg aaatgtatac aaatcaagat cgttatcaga 4320
gtccagaaaa taaagcccta ctggaaaata taaagcaggc tgtgagagga attcaggtct 4380
gaacagctgc tgtagtgatg aaactcttgc ttaaaaagga tgcctcttgt tttttgctgc 4440
tgtaacttac cagaaagtgt tctatattta tttctgtttg aatttgaaac agtgttatgc 4500

ttacaagact tcataatgat tttatgtctt gctttaaaga tagtacctgc agaatagttt 4560
ttgaatacac ccacattttg tacgtttcca tgtaagctga catagtgttc tgccatgtaa 4620
tgtttatagc tgctgatgta tgcacatttg ggggtatatc tatttctgaa gaggtaagct 4680
gatcaaaata aatagagtgt aaattctttt taatgcttta gtgattaaat gttttagtat 4740
tttgaactga aatggacaca caaacacaca cacgcacaca cagacccaca gctttgaatg 4800
atcatgttgt ggctgagcag ccgcttttta gacgttatca ttttgcctca tgttggagga 4860
ctttatggaa tttaagaaat acattttgtg tgcatattgt ttcatagcaa gaattcgttg 4920
caaaaatgct ttatttttga acaatgcttg gaaatattat gtgacttttt tgtttgtttg 4980
ttttaggagg atggtgtatg gtgggggcaa taaatgaggt tttttgcatt ccaaggaaat 5040
ggcatatgga ttaactgtaa gaaatgaaat aagtaattta ttgtaagaca acatcaagcc 5100
atggaaactt ggcagaagat tcaaagcagc ttaaacagca cttttaaatt aactcctaag 5160
cgttacatgg ttgtgactat ggaaactcca gttaagacag gatcttatca gaggtggaca 5220
acgtgaagat ttccttttcc attttcaata aactttggaa caaccttctc gtatctcccc 5280
tagagtttcg tgcccctctg aactgtctgt tattgcaatg tagtttatca acagaatttg 5340
tgtgttttcg atttaagcta aaagataatt taagaacatt tatttcccct tttcacttta 5400
aaaaattatg attattccta ttattgttat gaaccttctt attttacatt tgagggataa 5460
aggcaaatga tttgtgagtc ttctagttac tggaccgagt tttctgctgg atctggtggg 5520
aaggcagctc ggtaaagttt ccctcctgct ccccccgccc gactttgact ctgaatcagc 5580
atttggtcct attcagagga ctcttaccac gacgtttctg ttctacactt gggtggagac 5640
cagttgacca tagagcattt gcagagcctc attgtttgat ttcttgtgac tattctaaga 5700
atgaatgcaa tcagatttta aaagtaacta aatatacttc agcacttttt tgctttaaac 5760
tagatcatct tagacttgtt tataccttcc agatttgatt gttttactcc caatgactgc 5820
actatatgta tgcataagac cacttttgag cgctgtgttc ccccttctga gtagtccttt 5880
gacgacgtgt tgtgttttct gatgttgact tgagttccat ttagtagcat ctcttccttc 5940
catgtcttga tgttatgcag gaagtacaga cgtactttaa atttttgtta tgaaataaaa 6000
aaaagatggg ttttgtaaaa ataaaaaaaa aatattttta gcagaacagg acttacaggg 6060
tcattgtccc cacaatgtgc cagtcgacta tttgcactta ccttgtccta tatatccgta 6120
cggaggtgtg caattcctcg tgtcagtagc cttgtgacac tgaacctgga tggattatag 6180
aggagccctc acggctgatc aataatgttg caaagggaga ctacagggat ctcacgacga 6240
atattctgat acaatactca acctcggtat atatatatgt gtataaatat atgtatatcc 6300
cagcggcact ttatactgtt cactgtacaa aagcttacag ttttccacaa ggactttaat 6360
aactagctgg gaaaagacga tgtaattatt tcggggctct gcggaacctt ctctgtacag 6420
cgcccccttt ctgttgtgct attggttgca gctgccatgc tcagaatgcg ttttgagagc 6480
tgaagcaagg tgcttgcagt cacctgaggc cgtccgtgtg gcccagggcc ccagctgcct 6540
ttagggcccc cattgttcat aacagcatat gcatttcccc accgcgttgt gtctgcagct 6600
tctttgccaa tatagtaatg cttttagtag agtactagat agtatcagtt ttggattctt 6660
attgttatca cctatgtaca atggaaaggg attttaagca caaacctgct gctcatctaa 6720
cgttggtaca taatctcaaa tcaaaagtta tctgtgacta ttatataggg atcacaaaag 6780
tgtcacatat tagaatgctg acctttcata tggattattg tgagtcatca gagtttatta 6840
taacttattg ttcatattca tttctaagtt aatttaagta atcatttatt aagacagaat 6900
tttgtataaa ctatttattg tgctctctgt ggaactgaag tttgatttat ttttgtacta 6960
cacggcatgg gtttgttgac actttaattt tgctataaat gtgtggaatc acaagttgct 7020
gtgatacttc atttttaaat tgtgaacttt gtacaaattt tgtcatgctg gatgttaaca 7080
catcttactc taaataaaca aggtgttgcc acatttgtag cacgaaggat ctctaaaaaa 7140
aaaaaaaaaa aa 7152

<210> 9
<211> 2492
<212> DNA
<213> Artificial Sequence

<220>
<223> Inducing factor

<400> 9

ggcagacaat cgtaaagctg ctcgggggtc gctcacctgc tcccgcctcc tggagcccct 60
accgggtctg accgctcttg ccccctccat tgcccgcccc ccgcccgagc cgggccagga 120
gccgagaggt ccgagaggcc cgggaaggat gcggccagca gctgcggtcc cgagccacgc 180
acggtgatcg cctcctccag gggaggaggc gcctagaccg tagcctcatt tcttgttttc 240
ctctccctcc ccctccccgt aattacattg gttgctggaa gggacccgga gagatagaca 300
gaggaggcgc ctcgcatccc cctctcgccc gccacccgtg ctctttcctg tcccgggtca 360
ggatgactgg agtctttgac agtctggtgg ctgatatgca ctcgacccag atcaccgcct 420

ccagcacgta ccaccagcac cagcagcccc cgagcggtgc gggcgccggc cctggcggca 480
acagcaacag cagcagcagc aacagcagcc tgcacaagcc ccaggagtcg ccaaccctcc 540
cggtgtccac ggctacggac agcagctact acaccaacca gcagcacccg gcgggcggcg 600
gcggcggggg ggcctcgccc tacgcgcaca tgggctccta ccagtaccac gccagcggcc 660
tcaacaatgt ctcctactcc gccaaaagca gctacgacct gggctacacc gccgcgtaca 720
cctcctacgc gccctacggc accagttcgt ctccggtcaa caacgagccg gacaaggaag 780
accttgagcc tgaaatccga atagtgaacg ggaagccaaa gaaagtccgg aaaccacgca 840
ccatctactc cagtttccag ctggcggccc ttcaacgacg cttccagaag acccagtatc 900
tggccctgcc agagcgagcc gagctggcgg cgtccctggg cctcacccaa actcaggtca 960
aaatctggtt ccagaaccgc cgatccaagt tcaagaagat gtggaaaagc ggcgagatac 1020
ccaccgagca gcaccctgga gccagcgctt ctcctccttg tgcctccccg ccggtctcgg 1080
cgccagcatc ctgggacttc ggcgcgccgc agcggatggc tggcggcggc ccgggcagcg 1140
gaggcggcgg tgcgggcagc tctggctcca gcccgagcag cgccgcctcg gcctttctgg 1200
gaaactaccc gtggtaccac caggcttcgg gctccgcttc acacctgcag gccacagcgc 1260
cacttctgca tccttcgcag actccgcagg cgcaccatca ccaccatcac caccaccacg 1320
caggcggggg cgccccggtg agcgcgggga cgattttcta acctcgagtg gacagcgtct 1380
gagacttgag aactgagaca ccgtgttctt cagcacgcgt cctgtagcta gaggctcgaa 1440
cctaccagcc cgcgaccgcc acccacccag cggtattgga agtggcgacc aggggtgacc 1500
cgtcgtggag ccacttctcg cactgccacg gagcagccgg aaacaaggag gcctcgtggg 1560
atcctttagt gccaagcctg gcgtccctgg cctcaattcc gacctgagcc ccggccactt 1620
ttaggccatc ctcggacttg tcgccccttc ccagccccct cagcgtgatc ctcagggtcc 1680
ttggtctctt caggccctga gcggggtcag gcactgggac gccttccccg ccctcgaggc 1740
ttcccggctc tcagaggctc cttttttctc tcctgctcca ggcctcagct ccagtccgct 1800
tcggttattg acctcctggg tcctactcca ccctatcacc tacccccgtg acccccggga 1860
tcccgccggt tttttcccct cctgggggct catgggggag aattcccagc tctcccaact 1920
cttcccgcct cccactttcc ccaggaacac tcccgtagct ccttcatcca ttgccagtgg 1980
agggttttta tttttatttt tttaaaagtt taggtgcctt tgcggatgac ctcattttga 2040
tgttaaaaaa aaactgattt ttttaatatg tggacgctgc aaaaaatgtg tttaaattat 2100
cttttttaag atgaattaag gactattagc tcgaacttcg atgcagagtt tgtaaataag 2160
ggtgtctgtg cagatttccc cacatttatt tgtataaaaa tttctcatct tttcagactt 2220
ttttgtaaac cgcccccgag ttgtgaaagc tgcgacgtag cagtgacctc agcagtccct 2280
atgttttccc cctttgaaaa tttttctcag ttaaattaag ctactgattt gaatttgttt 2340
ttatcttgtc ctaaagtctt tgttcttgaa atgcaaggta tttgggggtt atttattatg 2400
aaagcaacat actcttaatg ttgattttac aatatgaaga gattatttaa aataaattat 2460
tgttttcatt ggaaaaaaaa aaaaaaaaaa aa 2492

<210> 10
<211> 2308
<212> DNA
<213> Artificial Sequence

<220>
<223> Inducing factor

<400> 10

aagaggatgc gaccagaggc agctgtcccg agcagcgcac ggtgatcgcc tcccccggag 60
gaggaggcgc ctagacggtt gcctcctttc ttgtttcccc accaccaccc ccgtaattac 120
attggctgct ggaggggacc gggagagaca gaggaggcgc ctggcttccc ccgcacgccc 180
gccacctctg ctctttcccg tctcgggcca ggatgactgg agtctttgac agtctagtgg 240
ctgatatgca ctcgacccag atcgccgcct ccagcacgta ccaccagcac cagcagcccc 300
cgagcggcgg cggcgccggc ccgggtggca acagcagcag cagcagcagc ctccacaagc 360
cccaggagtc gcccaccctt ccggtgtcca ccgccaccga cagcagctac tacaccaacc 420
agcagcaccc ggcgggcggc ggcggcggcg ggggctcgcc ctacgcgcac atgggttcct 480
accagtacca agccagcggc ctcaacaacg tcccttactc cgccaagagc agctatgacc 540
tgggctacac cgccgcctac acctcctacg ctccctatgg aaccagttcg tccccagcca 600
acaacgagcc tgagaaggag gaccttgagc ctgaaattcg gatagtgaac gggaagccaa 660
agaaagtccg gaaaccccgc accatctact ccagtttcca gctggcggct cttcagcggc 720
gtttccaaaa gactcaatac ttggccttgc cggagcgagc cgagctggcg gcctctctgg 780
gcctcaccca gactcaggtc aaaatctggt tccagaaccg ccggtccaag ttcaagaaga 840
tgtggaaaag tggtgagatc ccctcggagc agcaccctgg ggccagcgct tctccacctt 900
gtgcttcgcc gccagtctca gcgccggcct cctgggactt tggtgtgccg cagcggatgg 960
cgggcggcgg tggtccgggc agtggcggca gcggcgccgg cagctcgggc tccagcccga 1020

gcagcgcggc ctcggctttt ctgggcaact acccctggta ccaccagacc tcgggatccg 1080
cctcacacct gcaggccacg gcgccgctgc tgcaccccac tcagaccccg cagccgcatc 1140
accaccacca ccatcacggc ggcgggggcg ccccggtgag cgcggggacg attttctaac 1200
cccagggaga actcgccaga gactgagagc agagaccact tatcctcatt gcttaccccg 1260
agccggggtt ccctcctccc ggcccgctgc cgccacccac ctctcctgca ggctgcgacc 1320
tgcagtggcc cgtctcaggc cctgctcact cccggggcca ccaaacgggc ccctctctcg 1380
ggggaaccgg acagcagctt ggcaaaggcc tccctaaaag gccgcatttc tgacctgagc 1440
cccgggtctc ggctgtttcg agccccgcct cggacttgcc ttccctcccc tccgggtgag 1500
cctgtctggc gccttcctcg ccccgggctg agagctgggt cccgggagat ggaagcctcc 1560
caggcgcgcg aggcttcccg ggcgctctga ggcttctttc tcctcgcccg ctcccctggg 1620
ctcagctcgg acgctgcagt tattgacctc ccggtcccgc ctgcccgccc tcccccacgt 1680
ggccccttga cccgggcggc cccgccgctt ctttccttcc tgcagttccc agccctcgga 1740
gcccccatcc cttatcttac ccccaccgcg ctcccccagg agcgctccct cagctctctc 1800
ctcatccatc accagtggag tttttttatt tgttattttt ttaaaagttt aggtgccttt 1860
gcggatgacc tcattttgac gttgaaaaaa tgatttttta atatgtgaac actgcaaaaa 1920
tgtgtttaaa ttatcttttt taaaacctat tcaggattat tagcctggac ttggacacag 1980
agtttgtaaa taaaggtgtc tgtgcagatt ttcccactga tttatttgta taaaaatact 2040
catcttttca gacttttttg taaaccccca gttgtgaaaa ctgcagttta gcagtgacct 2100
cagcaacccc tcctttttat tttttccttt aaaaacattt cagttaaatt aagctactga 2160
tttggatttg ttttatcgta tcctaaagtc tttgttgttg aaatgaaagg tattttgggg 2220
ttatttatta tgaaaacaac atgctcttaa tgttgatttt acaatatgaa gagattattt 2280
aaataaatta ttgttttcat tggaaaaa 2308

## Description

### FIELD

The present invention relates to cell technology, and to a nerve cell production method.

### BACKGROUND

Induced pluripotent stem cells (iPS cells) are capable of being transformed into numerous types of cells composing the body. Therefore, iPS cells capable of being transformed into various types of somatic cells or tissues are considered promising for use in cell graft therapy and innovative drug development and research. In 2014, for example, retina cells produced from iPS cells were successfully applied in transplantation therapy. Projects are being pursued not only in Japan but throughout the world for creating brain cells and different organ cells from iPS cells for use in transplantation therapy.

Numerous methods for transforming iPS cells to differentiated cells exist in the prior art. For utilization of iPS cells in transplantation therapy, however, it is important to establish highly efficient inducing methods for iPS cells. Specifically, it is necessary to establish techniques to be used for inducing differentiation of iPS cells to differentiated cells, improving the efficiency and precision of induction and ensuring that the functionality of the created differentiated cells is able to withstand transplantation therapy.

Methods for inducing differentiated cells from iPS cells or embryonic stem cells (ES cells) to somatic cells have conventionally included methods that imitate the process of development, by combining hormones or growth factors that are the determinants of the properties of the cells, as well as low molecular compounds, and varying their quantity ratios or concentrations with time. However, it is difficult to completely emulate the development process in vitro, and efficiency is also poor. Moreover, induction of human somatic cells requires a much longer inducing period than for mice, with conventionally 3 months or longer, for example, being necessary to prepare mature nerves.

Another problem is that inducing efficiency differs widely depending on the line of ES/iPS cells, while the properties of induced somatic cells are non-homogeneous. When chemical substances have actually been added to different multiple ES cell clones to create various types of cells, it has been demonstrated that certain clones exist that are readily induced to pancreas cells or that are readily induced to heart cells, and therefore that different clones have varying induction potencies (see NPL 1, for example). In addition, it has been demonstrated that when the method known as a serum-free suspension culture method (SFEBq method) is used, in which iPS cells are cultured in medium free of serum or of chemical substances that inhibit neuron induction, to produce neurons from iPS cells/ES cells, thereby producing neurons from dozens of lines of iPS cells, some of the iPS/ES cell clones present are difficult to transform into neurons (see NPL 2, for example).

Specifically, cells which are induced from human ES/iPS cells by methods utilizing hormones or chemical substances have been confirmed to be fetal-stage somatic cells in the initial stages. It is extremely difficult to induce to mature human somatic cells, and their culturing requires long periods of several months. However, for drug development and transplant medicine for fully developed individuals, it is important to prepare somatic cells that match the maturation level of the individual.

For neurons, which include cells of a variety of different subtypes, it is not possible to induce to neuronal subtypes in a uniform manner from ES/iPS cells by methods utilizing hormones or chemical substances. Therefore, drug screening specific for designated neuronal subtypes is not possible. This lowers the efficiency for drug screening. For transplant medicine as well, it is not possible to concentrate and transplant only specific diseased cells.

For this reason, methods have been proposed wherein genes for the properties of specific somatic cells are directly transferred into ES/iPS cells using deoxyribonucleic acid (DNA) viruses which are integrated into the genome, to create the desired somatic cells. Methods using deoxyribonucleic acid (DNA) viruses which are integrated into the genome allow specific generation of mature neurons in very short time periods compared to methods using hormones or chemical substances, such as 2 weeks, for example. Moreover, generating neurons by specific gene transfer allows excitatory nerves alone, for example, to be obtained in a homogeneous manner. Therefore, specific drug screening for specific neuronal subtypes becomes possible, potentially making it possible to concentrate and transplant only cells specific to a disease, for transplant medicine.

However, in methods of inducing differentiation of stem cells to somatic cells using deoxyribonucleic acid (DNA) viruses which are integrated into the genome to cause expression of specific genes, the genes are inserted in the genome of the ES/iPS cells, causing damage to the endogenous genes. This has resulted in problems such as failure to properly accomplish drug screening, and the risk of canceration of grafts (see NPLs 3 and 4, for example).

### [Citation List]

### [Non-patent literature]

NPL 1: Nature Biotechnol. 26(3): 313-315, 2008.
NPL 2: PNAS, 111:12426-12431,2014
NPL 3: N Eng J Med, 346:1185-1193, 2002
NPL 4: Science 302: 415-419, 2003

### SUMMARY

### [TECHNICAL PROBLEM]

It is an object of the present invention to provide a nerve cell production method that allows nerve cells to be produced efficiently in a short period of time, without damaging cellular genes.

### [SOLUTION TO PROBLEM]

According to one aspect of the present invention there is provided a nerve cell production method that includes preparing stem cells, and introducing Sendai virus by infection to the stem cells to induce the stem cells into nerve cells by allowing the Sendai virus to express messenger ribonucleic acid (mRNA) that synthesizes inducing factors in the stem cells.

In the nerve cell production method, the stem cells may be artificial pluripotent stem cells (iPS cells).

In the nerve cell production method, the stem cells may be embryonic stem cells (ES cells).

In the nerve cell production method, the inducing factors may include neurogenin (NGN). The inducing factors may include only neurogenin (NGN).

In the nerve cell production method, the inducing factors may include achaete-scute homolog (ASCL).

In the nerve cell production method, the inducing factors may include myelin transcription factor (MYT).

In the nerve cell production method, the inducing factors may include distal-less homeobox (DLX).

In the nerve cell production method, the nerve cells may be neurons, neural stem cells, or neural precursor cells. The neurons may be of one or more types selected from the group consisting of excitatory neurons, inhibitory neurons, dopamine-producing neurons, locomotor neurons, and cerebral neurons. The nerve cells may be oligodendrocyte precursor cells and oligodendrocytes.

In the nerve cell production method, the nerve cells may be positive for one or more of NGN gene, ASCL gene, MYT gene, and DLX gene.

In the nerve cell production method, the nerve cells may be positive for one or more selected from the group consisting of tyrosine hydroxylase (TH), TUJ1, BRN2, NGN, β-III Tubulin, MAP2, PSA-NCAM, vGLUT, SATB2, chAT, HB9, MUNC13, HOMER1, vGAT, and GAD65/67.

In the nerve cell production method, the Sendai virus may be allowed to express mRNA of a drug resistance gene in the stem cells.

In the nerve cell production method, the drug resistance gene may be one or more selected from the group consisting of puromycin resistance gene, blasticidin resistance gene, hygromycin resistance gene, and neomycin resistance gene.

The nerve cell production method may include selecting cells exhibiting drug resistance after infection of the stem cells with Sendai virus.

The nerve cell production method may include selecting cells exhibiting drug resistance of one or more selected from the group consisting of puromycin resistance, blasticidin resistance, hygromycin resistance, and neomycin resistance, after infection of the stem cells with Sendai virus.

In the nerve cell production method, the density of the stem cells at the infection with Sendai virus may be from 0.2×10⁵ cells/well to 1.0×10⁶ cells/well in the wells of a 12-well plate.

In the nerve cell production method, the multiplicity of infection (MOI) of Sendai virus may be from 0.1 to 100.0 per time.

In the nerve cell production method, the culture medium when the stem cells are infected with Sendai virus may be one selected from mTeSR^{R}1, TeSR2^{R}, and Stem Fit.

### [ADVANTAGEOUS EFFECTS OF INVENTION]

According to the invention, it is possible to provide a nerve cell production method that allows nerve cells to be produced efficiently in a short period of time, without damaging cellular genes.

### BRIEF DESCRIPTION OF DRAWINGS

Fig. 1 is a microscope photograph of the cells in Example 1.
Fig. 2 is a fluorescence microscope photograph of the cells fluorescent-labeled using anti BRN2 antibodies in Example 1.
Fig. 3 is a fluorescence microscope photograph of the cells fluorescent-labeled using anti HOMER1 antibodies in Example 2.
Fig. 4 is a fluorescence microscope photograph of the cells fluorescent-labeled using anti MAP2 antibodies in Example 3.
Fig. 5 is a fluorescence microscope photograph of the cells fluorescent-labeled using anti MUNC13-1 antibodies in Example 4.
Fig. 6 is a fluorescence microscope photograph of the cells fluorescent-labeled using anti SATB2 antibodies in Example 5.
Fig. 7 is a fluorescence microscope photograph of the cells fluorescent-labeled using anti TUJ1 antibodies in Example 6.
Fig. 8 is a fluorescence microscope photograph of the cells fluorescent-labeled using anti vGLUT antibodies in Example 7.
Fig. 9 is a fluorescence microscope photograph of the cells fluorescent-labeled using anti chAT antibodies in Example 8.
Fig. 10 is a fluorescence microscope photograph of the cells fluorescent-labeled using anti HB9 antibodies in Example 9.
Fig. 11 is a fluorescence microscope photograph of the cells fluorescent-labeled using anti TH antibodies in Example 10.
Fig. 12(a) and 12(b) are microscope photographs of the cells in Example 11. Fig. 12(c) is a fluorescence microscope photograph of the cells fluorescent-labeled using anti TUJ1 antibodies in Example 11.
Fig. 13 is a fluorescence microscope photograph of the cells fluorescent-labeled using anti BRN2 antibodies in Example 12.
Fig. 14 is a fluorescence microscope photograph of the cells fluorescent-labeled using anti GAD65/67 antibodies in Example 13.
Fig. 15 is a fluorescence microscope photograph of the cells fluorescent-labeled using anti MAP2 antibodies in Example 14.
Fig. 16 is a fluorescence microscope photograph of the cells fluorescent-labeled using anti vGAT antibodies in Example 15.
Fig. 17 is a fluorescence microscope photograph of the cells fluorescent-labeled using anti TUJ1 antibodies in Example 16.
Fig. 18 is a fluorescence microscope photograph of the cells fluorescent-labeled using anti MAP2 antibodies in Example 17.
Fig. 19 is a fluorescence microscope photograph of the cells fluorescent-labeled using anti BRN2 antibodies in Example 18.
Fig. 20 is a fluorescence microscope photograph of the cells fluorescent-labeled using anti vGAT antibodies in Example 19.
Fig. 21 is a fluorescence microscope photograph of the cells fluorescent-labeled using anti GAD65/67 antibodies in Example 20.
Fig. 22 is a fluorescence microscope photograph of the cells fluorescent-labeled using anti TH antibodies in Example 21.
Fig. 23 is a fluorescence microscope photograph of the cells fluorescent-labeled using anti chAT antibodies in Example 22.
Fig. 24 is a fluorescence microscope photograph of the cells fluorescent-labeled using anti vGLUT antibodies in Example 23.
Fig. 25 is a fluorescence microscope photograph of the cells fluorescent-labeled using anti SATB2 antibodies in Example 24.
Fig. 26 is a graph showing the results of flow cytometry in Example 25.

### DESCRIPTION OF EMBODIMENTS

Embodiments of the invention will now be explained in detail. The embodiments described below are merely examples of devices and methods for implementing the technical concept of the invention, and the technical concept of the invention is not limited to the described combinations of structural components. The technical concept of the invention may incorporate various modifications such as are within the scope of the Claims.

The method of producing somatic cells from stem cells according to the embodiments of the invention includes preparing stem cells, and introducing Sendai virus by infection to the stem cells to induce the stem cells into nerve cells by allowing the Sendai virus to express mRNA that synthesizes inducing factors in the stem cells.

Both induced pluripotent stem cells (iPS cells) and embryonic stem cells (ES cells) can be used as stem cells. The stem cells may be human stem cells or non-human animal stem cells.

Induction refers to reprogramming, initialization, transformation, differentiation transformation (Transdifferentiation or Lineage reprogramming), differentiation induction, cell f ate reprogramming, and the like.

Examples of nerve cells to be induced include neurons, neural stem cells and neural precursor cells. Examples of neurons include excitatory neurons, inhibitory neurons, dopamine-producing neurons, locomotor neurons, and cerebral neurons. Alternatively, the nerve cells may be oligodendrocyte precursor cells, oligodendrocytes, and the like.

The Sendai virus (SeV) introduced into the stem cells has an RNA genome which expresses mRNAs to synthesize inducing factors which induce stem cells into neurons. Incidentally, production of recombinant Sendai virus vectors capable of expressing any arbitrary mRNA may be commissioned to, for example, ID Pharma Co., Ltd. using patented technology. mRNAs for synthesizing inducing factors include, for example, mRNAs of NGN genes such as NGN2, mRNAs of ASCL genes such as ASCL1, mRNAs of MYT genes such as MYT1L, and mRNAs of DLX genes such as DLX2. NGN2, ASCL1, MYT1L and DLX2 are switching proteins required for generating neurons. NGN2 is capable of generating excitatory neurons. Each of ASCL1, MYT1L, or DLX2 is capable of generating inhibitory neurons. The combination of ASCL1 and MYT1L is capable of generating excitatory neurons, inhibitory neurons, and dopamine-producing neurons.

For example, the Sendai viruses introduced into the stem cells express one or more selected from mRNAs of NGN genes such as NGN2, mRNAs of ASCL genes such as ASCL1, mRNAs of MYT genes such as MYT1L, and mRNAs of DLX genes such as DLX2, as mRNAs synthesizing inducing factors.

The Sendai viruses may have an RNA genome expressing mRNAs of drug resistance genes. Examples of drugs include antibiotics such as puromycin, blasticidin, hygromycin, neomycin, G418, and zeocin. For example, the Sendai viruses introduced into the stem cells express mRNAs of drug resistance genes. The cells in which mRNAs of drug resistance genes are expressed exhibit drug resistance.

Cells which exhibit drug resistance after infection may be selected when the Sendai viruses are allowed to express mRNAs of drug resistance genes. For example, when one or more of the mRNAs of puromycin-resistant genes, blasticidin-resistant genes, hygromycin-resistant genes, neomycin-resistant genes, G418-resistant genes, and zeocin^{R}-resistant genes are expressed by Sendai viruses, cells in which the Sendai viruses are introduced can be screened by exposing the cells after infection to the corresponding antibiotic and killing the cells other than the ones into which the Sendai viruses have been introduced.

The Sendai viruses may have, for example, RNAs capable of expressing mRNAs including mRNAs of NGN2 gene and mRNAs of puromycin resistance gene (hereinafter referred to as "NGN2-Puro mRNA(s)"). The sequence of the mRNAs of NGN2 gene corresponds, for example, to the DNA sequence shown as SEQ ID NO.1 (murine) or SEQ ID NO.2 (human). The cells in which NGN2-Puro mRNAs are expressed produce NGN2 and exhibit puromycin resistance.

The Sendai viruses may have, for example, RNAs capable of expressing mRNAs including mRNAs of ASCL1 gene, mRNAs of MYT1L gene, and mRNAs of puromycin resistance gene (hereinafter referred to as "ASCLI-MYTIL-Puro mRNA(s)"). The sequence of the mRNAs of ASCL1 gene corresponds, for example, to the DNA sequence shown as SEQ ID NO.3 (murine) or SEQ ID NO.4 (human). The sequence of the mRNAs of MUT1L gene corresponds, for example, to the DNA sequence shown as SEQ ID NO.5 (murine), SEQ ID NO.6 (murine), SEQ ID NO.7 (murine), or SEQ ID NO.8 (human). The cells in which ASCLI-MYTIL-Puro mRNAs are expressed produce ASCL1 and MYT1L and exhibit puromycin resistance.

The Sendai viruses may have, for example, RNAs capable of expressing mRNAs including mRNAs of DLX2 gene and mRNAs of blasticidin resistance gene (hereinafter referred to as "DLX2-Blast mRNA(s)"). The sequence of the mRNAs of DLX2 gene corresponds, for example, to the DNA sequence shown as SEQ ID NO.9 (murine) or SEQ ID NO.10 (human). The cells in which DLX2-Blast mRNAs are expressed produce DLX2 and exhibit blasticidin resistance.

The Sendai viruses are introduced into the stem cells by being suspended in the cell culture medium. The Sendai viruses recognize the cell surface antigens and infects the stem cells.

The density of the stem cells at the infection with Sendai virus is, for example, from 0.2×10⁵ cells/well to 1.0×10⁶ cells/well, from 0.5×10⁵ cells/well to 8.0×10⁵ cells/well, or from 1.0×10⁵ cells/well to 4×10⁵ cells/well, in the wells of a 12-well plate.

The titer of the Sendai viruses used is, for example, from 1×10¹² CIU/mL to 1×10⁵ CIU/mL, from 1×10¹⁰ CIU/mL to 1×10⁶ CIU/mL, or from 1×10⁹ CIU/mL to 1×10⁷ CIU/mL. The multiplicity of infection (MOI) of the Sendai viruses is, for example, from 0.1 to 100.0, from 1.0 to 50.0, or from 1.0 to 20.0 per time.

The culture medium used at the infection with Sendai virus is, for example, stem cell culture medium such as mTeSR1^{R}, TeSR2^{R} (STEMCELL Technologies Inc.), and Stem Fit (ReproCELL Inc.). After infection with Sendai virus, the culture medium may be replaced with a culture medium suitable for induction to nerve cells (hereinafter also referred to as "neural induction culture medium"). Examples for a neural induction culture medium include NDiff 227^{R} (TAKARA BIO INC.), Neurobasal^{R} (Thermo Fisher Scientific Inc.), Human ES/iPS Neuronal Differentiation Medium (Merck KGaA), and N3 culture medium. N3 culture medium is prepared, for example, by adding 10 mL of B27, 5 mL of N2 Supplement (Thermo Fisher Scientific Inc.), and 1.6 mL of insulin at a concentration of 6.25 mg/mL to 500 mL of DMEMF12.

The culture medium used during, before, and after the infection with Sendai virus may contain B18R proteins. B18R proteins alleviate the innate antiviral response of the cells. B18R proteins may be used to inhibit cell-death associated with immune reactions associated with viral infection. However, since the method according to the embodiments is capable of producing nerve cell from the stem cells in a short period of time, the culture medium may not contain B18R proteins, or may contain B18R proteins in a low concentration of such as 0.01% to 1%.

The stem cells are induced into nerve cells within 25 days, 20 days, 10 days, 9 days, 8 days, 7 days, 6 days, 5 days, or 4 days from the infection with Sendai virus. Whether or not the stem cells are induced to nerve cells is, for example, confirmed by the positivity or negativity of one or more selected from MAP2, TUJ1 (β-III Tubulin), BRN2, HOMER1, NGN such as NGN2, PSA-NCAM, MUNC13-1, SATB2, vGLUT, chAT, HB9, LHX3, GAD65, GAD67, TH, ASCL such as ASCL1 (MASH1), vGAT, SOX1, SOX2, CD133, Nestin, HB9, ISL1, O4, PLP1, MOG, and MBP.

NGN2 is a switching protein required for producing neurons and is a marker for excitatory neurons. TUJ1 (β-III Tubulin), MAP2, BRN2, ASCL1 (MASH1), and PSA-NCAM are markers for neurons. HOMER1 and MUNC 13-1 are markers for mature neurons which form synapses. SATB2 is a marker for cerebrocortical neurons. vGLUT is a marker for excitatory neurons such as glutamatergic neurons. chAT is a marker for locomotor neurons such as cholinergic neurons. HB9 is a marker for locomotor neurons. GAD65 and GAD67 are markers for inhibitory neurons such as GABAergic neurons. TH is a marker for dopamine-producing neurons. vGAT is a marker for inhibitory neurons. SOX1, SOX2, CD133 and Nestin are markers for neural stem cells. LHX3, HB9, and ISL1 are markers for locomotor neurons. O4, PLP1, MOG, and MBP are markers for oligodendrocyte precursors. GFAP and CD44 are also available as markers for astrocyte precursors and astrocytes.

According to the method of the embodiments of the invention described above, it is possible to efficiently produce nerve cells integration-free and without damaging the genes of the stem cells by allowing the stem cells to express the mRNAs synthesizing the inducing factors.

In a method for producing nerve cells from stem cells using only hormones and chemicals, it requires quite a long time so that nerve cells are produced. Whereas, in the nerve cell production method according to the embodiments of the invention, it is possible to produce nerve cells in a short period of time.

Moreover, in the method for producing nerve cells from stem cells using only hormones and chemicals, only a portion of the stem cells become the intended nerve cells. Whereas, in the nerve cell production method according to the embodiments of the invention, most of the cells in which infection with Sendai virus has been established can become the intended nerve cells.

Furthermore, in the method for producing nerve cells from stem cells using only hormones and chemicals, there are clones that become the intended nerve cells and clones that do not although through the same protocol, and variation occurs among the clones. Whereas, in the method according to the embodiments of the invention, it is possible to obtain a high induction efficiency in a plurality of clones.

Moreover, when producing cells for transplantation through induction using cytokines from an undifferentiated cell population, there is a possibility that some uninduced cells remain in the cells for transplantation. Such remaining uninduced cells may independently expand through cell division at the transplantation site and form teratomas or the like. Whereas, in the nerve cell production method according to the embodiments of the invention, it is possible to allow simultaneous expression of mRNAs of drug resistance genes so that a drug selection of the cells into which Sendai virus has been introduced can be performed. Therefore, it is possible to avoid the risks such as contamination of uninduced cells and teratoma formation, and it is suitable for transplantation medicine.

Furthermore, in the method according to the embodiments of the invention, Sendai viruses are used and viruses which insert genes into the genomes are not used. Therefore, it is integration-free, does not damage the stem cell genes, and has no risks of cancerization in the produced nerve cells, making them available for clinical use.

Still further, cleaner and safer nerve cells can be produced by, for example, producing iPS cells from blood cells in a clean environment of a fully closed system, and subsequently producing the nerve cells from the iPS cells in a clean environment of a fully closed system by the method according to the embodiments of the invention.

Additionally, in the method according to the embodiments of the invention, since the nerve cells can be produced in a short period of time, it is not necessary to use B18R or the like, and it can be set at a very low concentration even in case of use.

### (Example 1)

A Sendai virus capable of expressing NGN2-Puro mRNA was prepared at ID Pharma Co., Ltd. The titer of the prepared Sendai virus was 1×10⁹ CIU/mL.

A 12-well dish coated with solubilized basement membrane preparation (Matrigel, Corning) was prepared, and a feeder-free culture medium (mTeSR^{R}1, Stemcell Technologies) containing ROCK (Rho-associated coiled-coil forming kinase/Rho-associated kinase) inhibitor (Selleck) at a concentration of 10 nmol/mL was added to each well. ROCK inhibitors inhibit cell death.

iPS cells were dispersed in detachment/dissociation/dispersion solution of tissues and cultured cells (Accutase, Innovative Cell Technologies) and seeded onto the 12-well dish. The iPS cells which are to be infected with Sendai virus were seeded at a density of 2×10⁵ cells/well. Control iPS cells which are not to be infected with Sendai virus were also seeded at a density of 2×10⁵ cells/well. Then, the iPS cells were cultured in feeder-free culture medium under the gas condition of 5% carbon dioxide concentration and 20% oxygen concentration for 24 hours.

The iPS cells were infected with Sendai virus capable of expressing NGN2-Puro mRNAs at a MOI of 20. On Day 2 after infection with Sendai virus, the culture medium in the wells was replaced with a neural induction culture medium (N3 culture medium) containing puromycin at a concentration of 2 µg/mL to kill the uninfected cells. The N3 culture medium has been prepared by adding 10 mL of B27, 5 mL of N2, and 1.6 mL of insulin with a concentration of 6.25 mg/mL to 500 mL of DMEMF12.

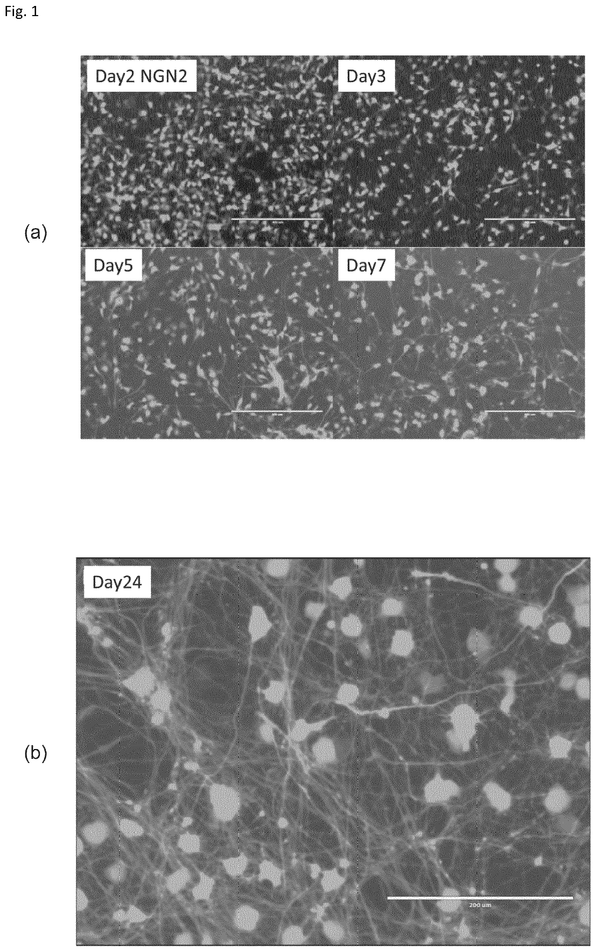

The microscope photographs of the cells on Day 2, Day 3, Day 5, and Day 7 after infection with Sendai virus are shown in Fig. 1(a). The microscope photograph of the cells on Day 24 after infection with Sendai virus is shown in Fig. 1(b). As shown in Fig. 1(a), induction of the cells to nerve cells after infection with Sendai virus has been morphologically confirmed.

On Day 28 after infection with Sendai virus, the culture medium was removed from the plate, and the cells were washed with PBS. Then, 4% paraformaldehyde (PFA) was added in the plate, and allowed to react for 15 minutes at 4 °C to fix the cells. The cells were further washed twice with PBS, and primary antibodies diluted with PBS culture medium containing 5% CCS and 0.1% Triton are added to the plate. As the primary antibodies, goat monoclonal antibodies (Santa Cruz Biotechnology) against BRN2 which is a marker for neurons were used.

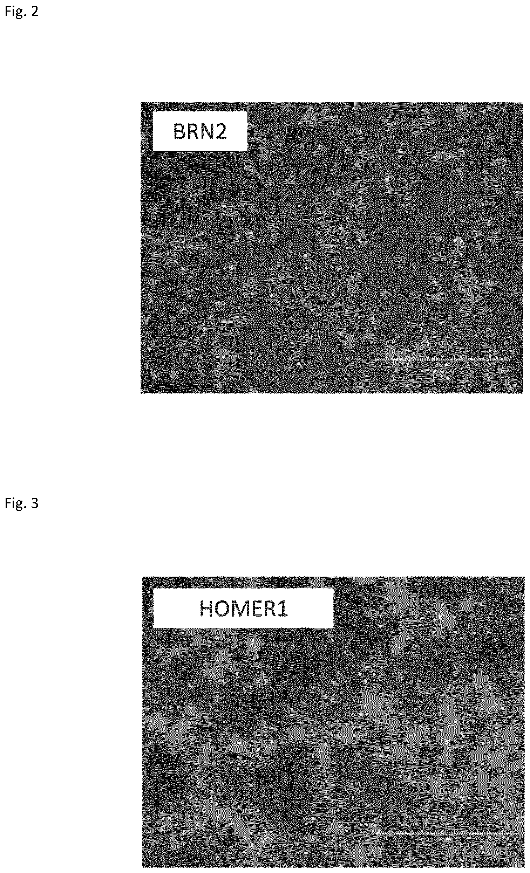

After allowing to react for 1hour at room temperature, PBS was added to the plate, thoroughly mixed, and then the PBS was discarded. PBS was again added and discarded, and a solution containing a fluorescent-labeled gorilla anti-goat IgG(H+L) secondary antibodies (Alexa Fluor^{R}, 555, conjugate, ThermoFisher) was added to the plate, and allowed to react for 30 minutes at room temperature. The cells were then washed twice with PBS and observed under a fluorescence microscope. As a result, it has been confirmed that the induced nerve cells expressed BRN2 as shown in Fig. 2.

### (Example 2)

In the same manner as in Example 1, iPS cells were infected with Sendai virus capable of expressing NGN2-Puro mRNAs, the cells were fluorescent-labeled using rabbit monoclonal antibodies (SYNAPTIC SYSTEMS) against HOMER1 which is a marker for mature neurons forming synapses as primary antibodies, and observed under a fluorescence microscope. As a result, it has been confirmed that the induced nerve cells expressed HOMER1 as shown in Fig. 3.

### (Example 3)

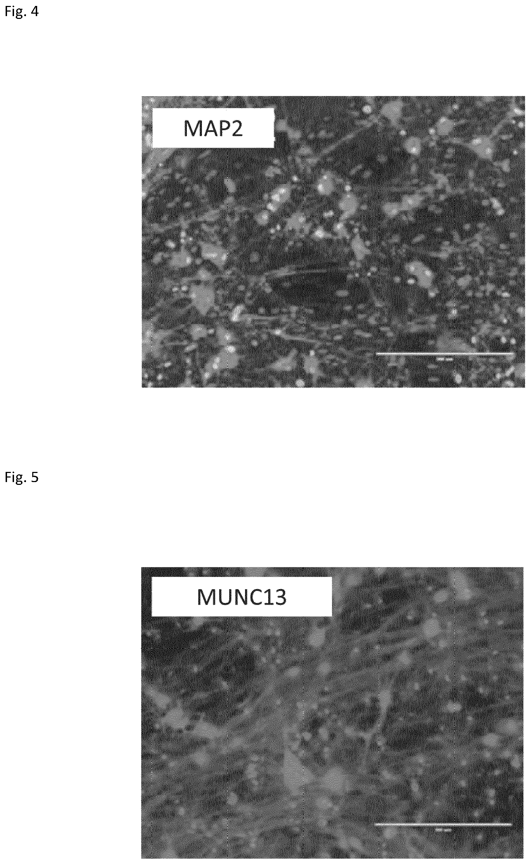

In the same manner as in Example 1, iPS cells were infected with Sendai virus capable of expressing NGN2-Puro mRNAs, the cells were fluorescent-labeled using mouse monoclonal antibodies (Sigma Aldrich) against MAP2 which is a marker for neurons as primary antibodies, and observed under a fluorescence microscope. As a result, it has been confirmed that the induced nerve cells expressed MAP2 as shown in Fig. 4.

### (Example 4)

In the same manner as in Example 1, iPS cells were infected with Sendai virus capable of expressing NGN2-Puro mRNAs, the cells were fluorescent-labeled using rabbit monoclonal antibodies (SYNAPTIC SYSTEMS) against MUNC13-1 which is a marker for mature neurons forming synapses as primary antibodies, and observed under a fluorescence microscope. As a result, it has been confirmed that the induced nerve cells expressed MUNC13-1 as shown in Fig. 5.

### (Example 5)

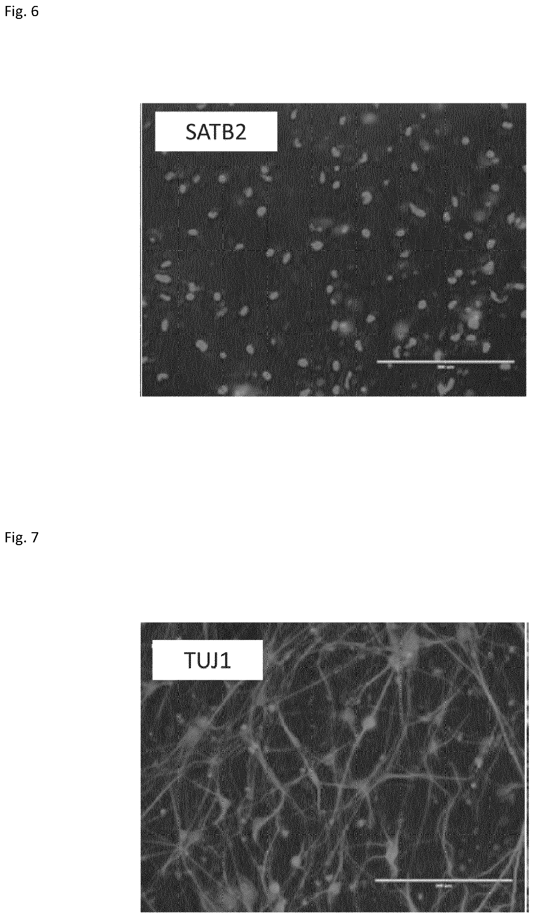

In the same manner as in Example 1, iPS cells were infected with Sendai virus capable of expressing NGN2-Puro mRNAs, the cells were fluorescent-labeled using mouse monoclonal antibodies (Abcam) against SATB2 which is a marker for cerebral neurons as primary antibodies, and observed under a fluorescence microscope. As a result, it has been confirmed that the induced nerve cells expressed SATB2 as shown in Fig. 6.

### (Example 6)

In the same manner as in Example 1, iPS cells were infected with Sendai virus capable of expressing NGN2-Puro mRNAs, the cells were fluorescent-labeled using mouse monoclonal antibodies (Biolegend) against TUJ1 which is a marker for neurons as primary antibodies, and observed under a fluorescence microscope. As a result, it has been confirmed that the induced nerve cells expressed TUJ1 as shown in Fig. 7.

### (Example 7)

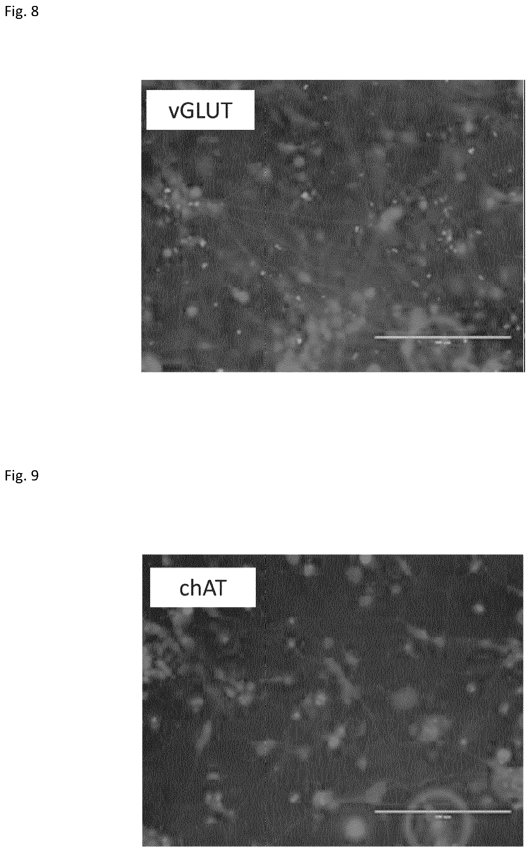

In the same manner as in Example 1, iPS cells were infected with Sendai virus capable of expressing NGN2-Puro mRNAs, the cells were fluorescent-labeled using rabbit monoclonal antibodies (SYNAPTIC SYSTEMS) against vGLUT which is a marker for excitatory neurons such as glutamatergic neurons as primary antibodies, and observed under a fluorescence microscope. As a result, it has been confirmed that the induced nerve cells expressed vGLUT as shown in Fig. 8.

### (Example 8)

In the same manner as in Example 1, iPS cells were infected with Sendai virus capable of expressing NGN2-Puro mRNAs, the cells were fluorescent-labeled using mouse monoclonal antibodies (Millipore) against chAT which is a marker for locomotor neurons as primary antibodies, and observed under a fluorescence microscope. As a result, it has been confirmed that the induced nerve cells expressed chAT as shown in Fig. 9.

### (Example 9)

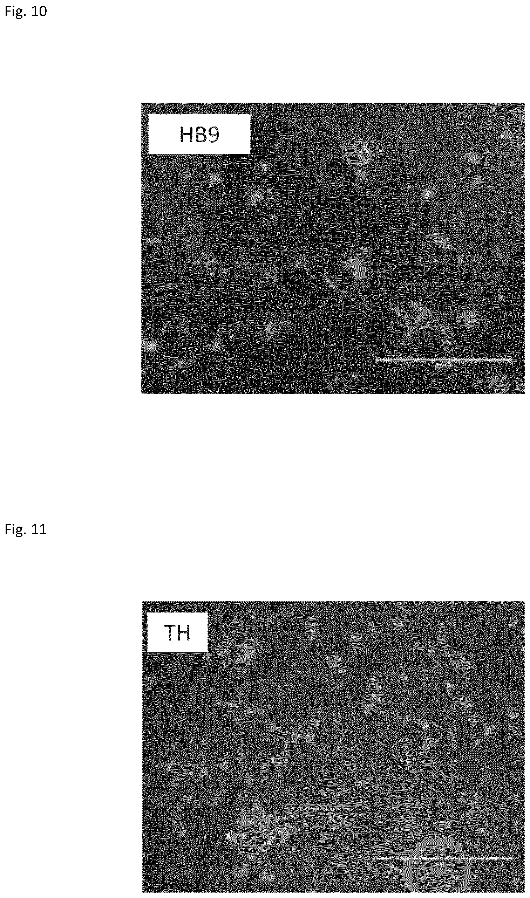

In the same manner as in Example 1, iPS cells were infected with Sendai virus capable of expressing NGN2-Puro mRNAs, the cells were fluorescent-labeled using mouse monoclonal antibodies (Thermo) against HB9 which is a marker for locomotor neurons as primary antibodies, and observed under a fluorescence microscope. As a result, it has been confirmed that the induced nerve cells expressed HB9 as shown in Fig. 10.

### (Example 10)

In the same manner as in Example 1, iPS cells were infected with Sendai virus capable of expressing NGN2-Puro mRNAs, the cells were fluorescent-labeled using sheep monoclonal antibodies (Pel Freez) against TH which is a marker for dopamine-producing neurons as primary antibodies, and observed under a fluorescence microscope. As a result, it has been confirmed that the induced nerve cells expressed TH as shown in Fig. 11.

### (Example 11)

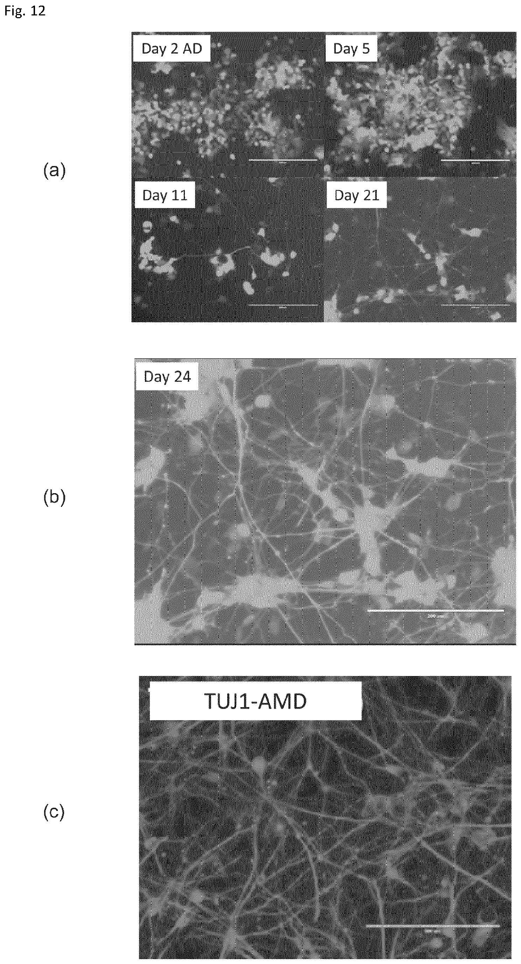

iPS cells were infected in the same manner as in Example 1 except that Sendai virus capable of expressing ASCL1-MYT1L-Puro mRNAs and DLX2-Blast mRNAs are used. The microscope photographs of the cells on Day 2, Day 5, Day 11, and Day 21 after infection with Sendai virus are shown in Fig. 12(a). The microscope photograph of the cells on Day 24 after infection with Sendai virus is shown in Fig. 12(b). As shown in Fig. 12(a) and Fig. 12(b), induction of the cells to nerve cells after infection with Sendai virus has been morphologically confirmed.

On Day 28 after infection with Sendai virus, the cells were fluorescent-labeled using mouse monoclonal antibodies (Biolegend) against TUJ1 which is a marker for average neurons as primary antibodies, and observed under a fluorescence microscope. As a result, it has been confirmed that the induced nerve cells expressed TUJ1 as shown in Fig. 12(c).

### (Example 12)

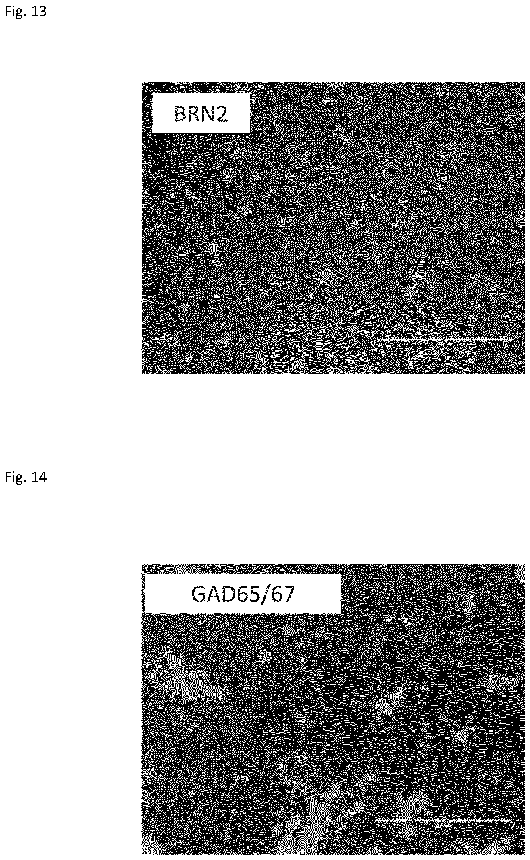

In the same manner as in Example 11, iPS cells were infected with Sendai virus capable of expressing ASCL1-MYT1L-Puro mRNAs and DLX2-Blast mRNAs, the cells were fluorescent-labeled using goat monoclonal antibodies (Santa Cruz Biotechnology) against BRN2 which is a marker for neurons as primary antibodies, and observed under a fluorescence microscope. As a result, it has been confirmed that the induced nerve cells expressed BRN2 as shown in Fig. 13.

### (Example 13)

In the same manner as in Example 11, iPS cells were infected with Sendai virus capable of expressing ASCL1-MYT1L-Puro mRNAs and DLX2-Blast mRNAs, the cells were fluorescent-labeled using mouse monoclonal antibodies (Millipore) against GAD65/67 which are markers for inhibitory (GABAergic) neurons as primary antibodies, and observed under a fluorescence microscope. As a result, it has been confirmed that the induced nerve cells expressed GAD65/67 as shown in Fig. 14.

### (Example 14)

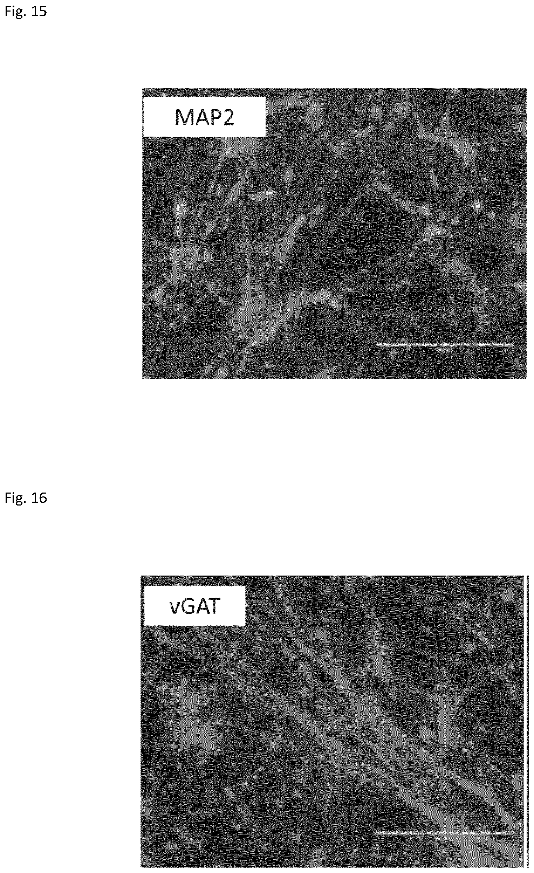

In the same manner as in Example 11, iPS cells were infected with Sendai virus capable of expressing ASCL1-MYT1L-Puro mRNAs and DLX2-Blast mRNAs, the cells were fluorescent-labeled using mouse monoclonal antibodies (Sigma Aldrich) against MAP2 which is a marker for neurons as primary antibodies, and observed under a fluorescence microscope. As a result, it has been confirmed that the induced nerve cells expressed MAP2 as shown in Fig. 15.

### (Example 15)

In the same manner as in Example 11, iPS cells were infected with Sendai virus capable of expressing ASCL1-MYT1L-Puro mRNAs and DLX2-Blast mRNAs, the cells were fluorescent-labeled using rabbit monoclonal antibodies (SYNAPTIC SYSTEMS) against vGAT which is a marker for inhibitory neurons as primary antibodies, and observed under a fluorescence microscope. As a result, it has been confirmed that the induced nerve cells expressed vGAT as shown in Fig. 16.

### (Example 16)

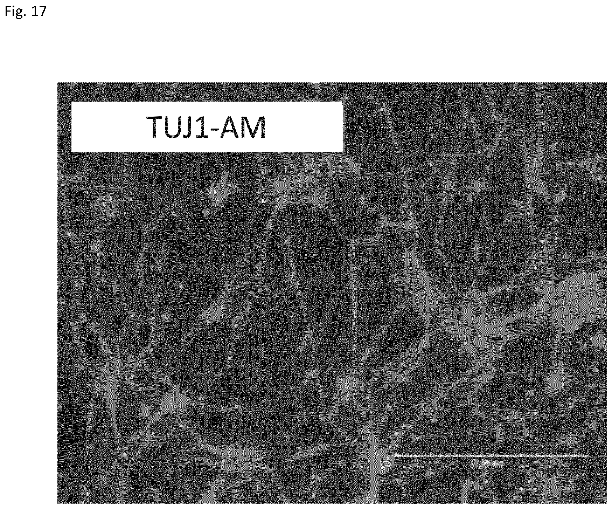

iPS cells were infected in the same manner as in Example 1 except that Sendai virus capable of expressing ASCL1-MYT1L-Puro mRNAs are used. On Day 21 after infection with Sendai virus, the cells were fluorescent-labeled using mouse monoclonal antibodies (Biolegend) against TUJ1 which is a marker for average neurons as primary antibodies, and observed under a fluorescence microscope. As a result, it has been confirmed that the induced nerve cells expressed TUJ1 as shown in Fig. 17.

### (Example 17)

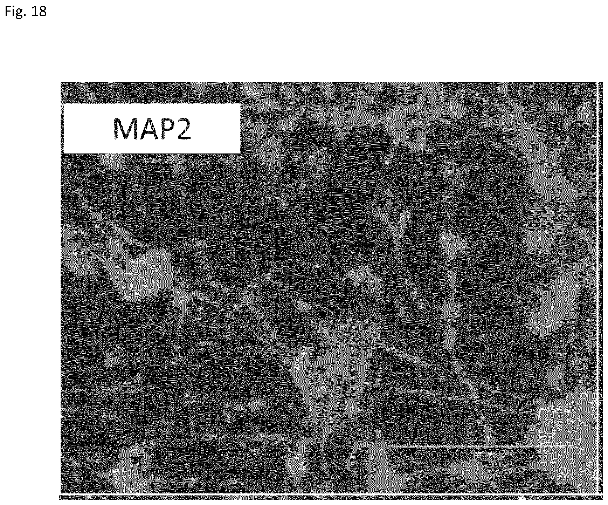

In the same manner as in Example 22, iPS cells were infected with Sendai virus capable of expressing ASCL1-MYT1L-Puro mRNAs, the cells were fluorescent-labeled using mouse monoclonal antibodies (Sigma Aldrich) against MAP2 which is a marker for neurons as primary antibodies, and observed under a fluorescence microscope. As a result, it has been confirmed that the induced nerve cells expressed MAP2 as shown in Fig. 18.

### (Example 18)

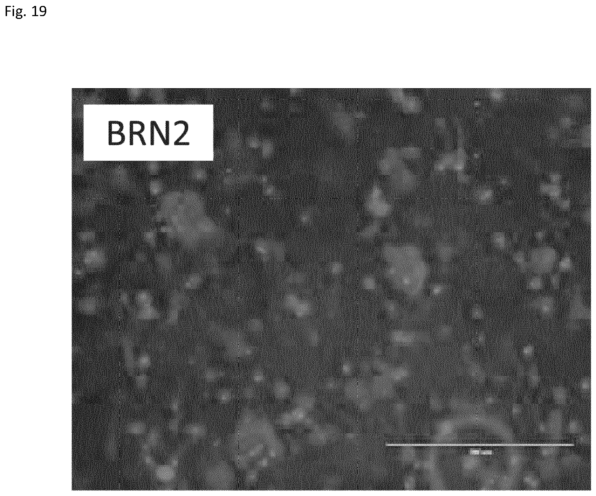

In the same manner as in Example 22, iPS cells were infected with Sendai virus capable of expressing ASCL1-MYT1L-Puro mRNAs, the cells were fluorescent-labeled using goat monoclonal antibodies (Santa Cruz Biotechnology) against BRN2 which is a marker for neurons as primary antibodies, and observed under a fluorescence microscope. As a result, it has been confirmed that the induced nerve cells expressed BRN2 as shown in Fig. 19.

### (Example 19)

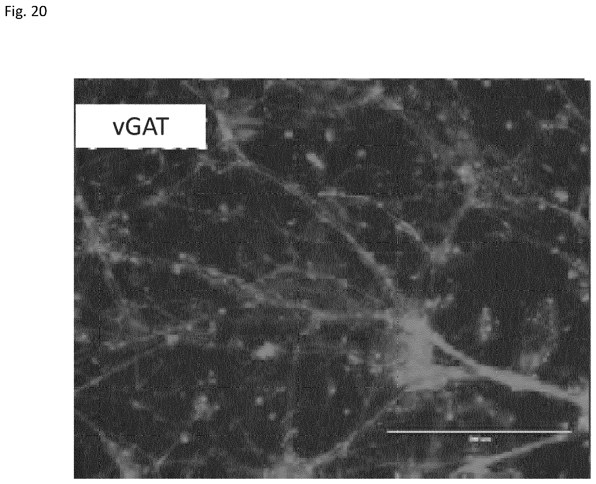

In the same manner as in Example 22, iPS cells were infected with Sendai virus capable of expressing ASCL1-MYT1L-Puro mRNAs, the cells were fluorescent-labeled using rabbit monoclonal antibodies (SYNAPTIC SYSTEMS) against vGAT which is a marker for inhibitory neurons as primary antibodies, and observed under a fluorescence microscope. As a result, it has been confirmed that the induced nerve cells expressed vGAT as shown in Fig. 20.

### (Example 20)

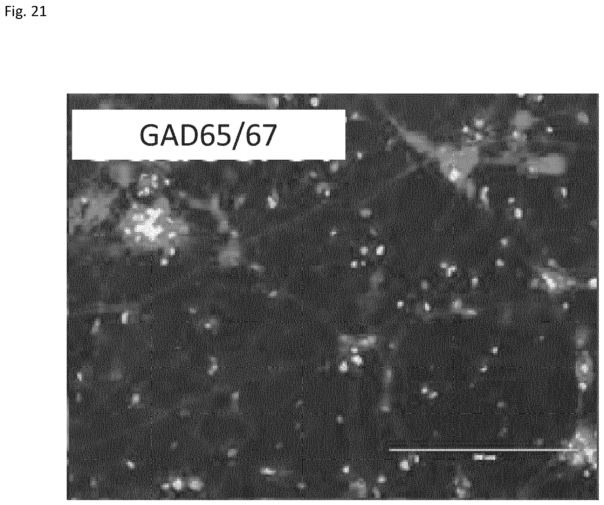

In the same manner as in Example 22, iPS cells were infected with Sendai virus capable of expressing ASCL1-MYT1L-Puro mRNAs, the cells were fluorescent-labeled using mouse monoclonal antibodies (Millipore) against GAD65/67 which are markers for inhibitory neurons (GABAergic neurons) as primary antibodies, and observed under a fluorescence microscope. As a result, it has been confirmed that the induced nerve cells expressed GAD65/67 as shown in Fig. 21.

### (Example 21)

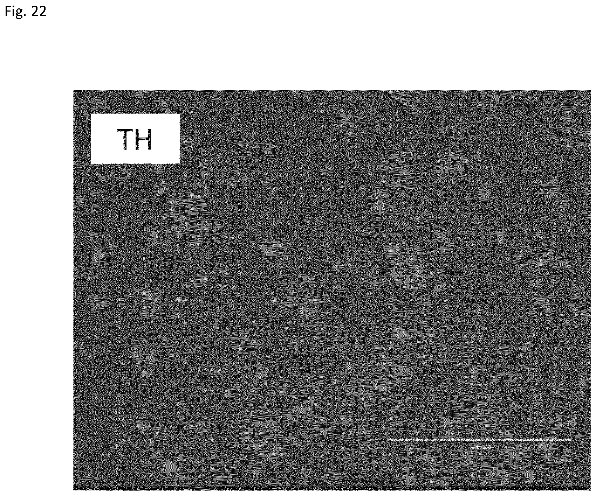

In the same manner as in Example 22, iPS cells were infected with Sendai virus capable of expressing ASCL1-MYT1L-Puro mRNAs, the cells were fluorescent-labeled using sheep monoclonal antibodies (Pel Freez) against TH which is a marker for dopamine-producing neurons as primary antibodies, and observed under a fluorescence microscope. As a result, it has been confirmed that the induced nerve cells expressed TH as shown in Fig. 22.

### (Example 22)

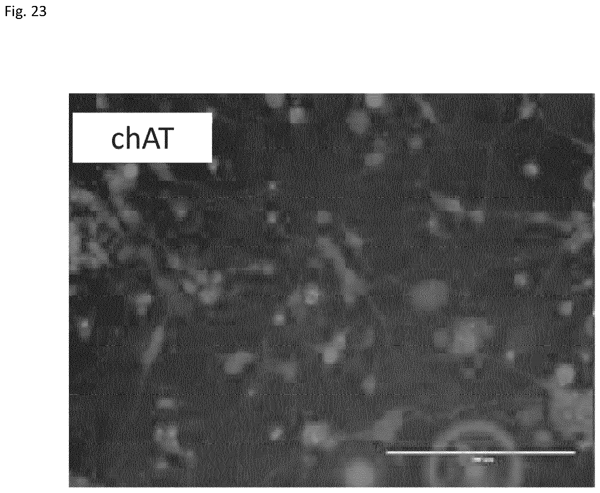

In the same manner as in Example 22, iPS cells were infected with Sendai virus capable of expressing ASCL1-MYT1L-Puro mRNAs, the cells were fluorescent-labeled using goat monoclonal antibodies (Millipore) against chAT which is a marker for cholinergic neurons as primary antibodies, and observed under a fluorescence microscope. As a result, it has been confirmed that the induced nerve cells expressed chAT as shown in Fig. 23.

### (Example 23)

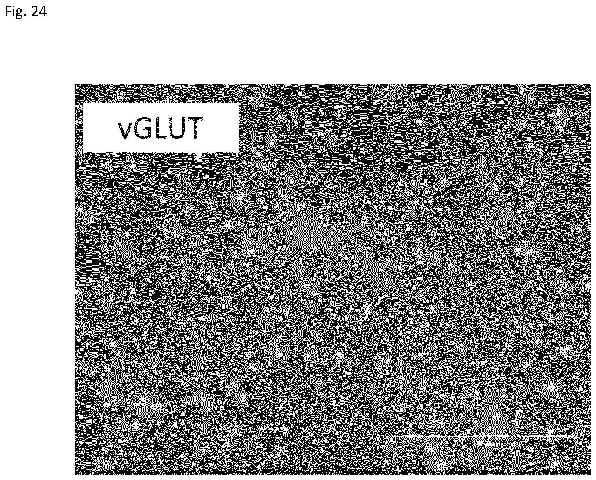

In the same manner as in Example 22, iPS cells were infected with Sendai virus capable of expressing ASCL1-MYT1L-Puro mRNAs, the cells were fluorescent-labeled using rabbit monoclonal antibodies (SYNAPTIC SYSTEMS) against vGLUT which is a marker for excitatory neurons (glutamatergic neurons) as primary antibodies, and observed under a fluorescence microscope. As a result, it has been confirmed that the induced nerve cells expressed vGLUT as shown in Fig. 24.

### (Example 24)

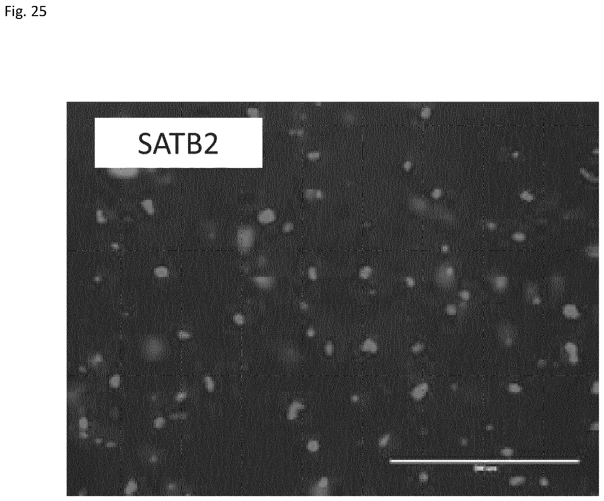

In the same manner as in Example 22, iPS cells were infected with Sendai virus capable of expressing ASCL1-MYT1L-Puro mRNAs, the cells were fluorescent-labeled using mouse monoclonal antibodies (Abcam) against SATB2 which is a marker for cerebral neurons as primary antibodies, and observed under a fluorescence microscope. As a result, it has been confirmed that the induced nerve cells expressed SATB2 as shown in Fig. 25.

### (Example 25)

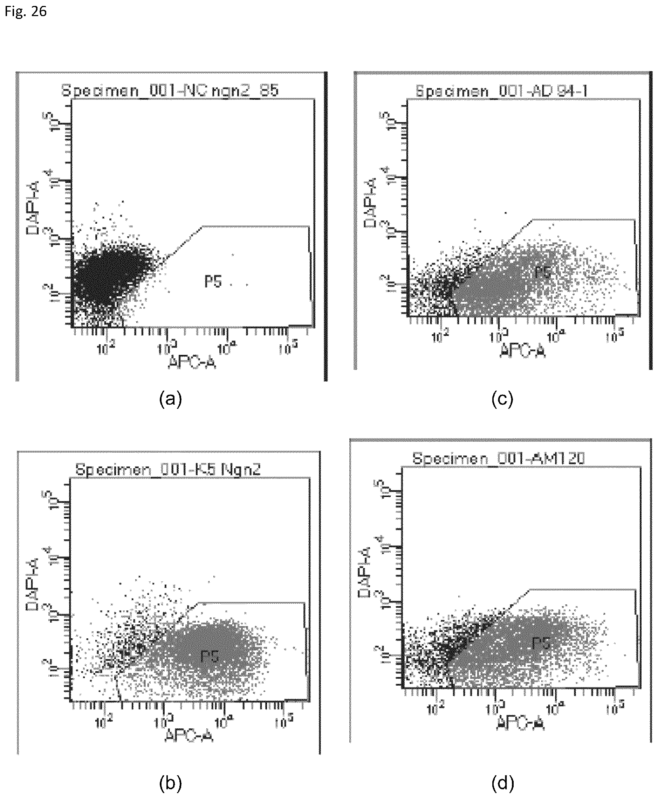

A flow cytometry analysis regarding the control cells which had not been infected with Sendai virus capable of expressing NGN2-Puro mRNAs prepared in Example 1 showed that the control cells were negative for PSA-NCAM which is a marker for neurons, as shown in Fig. 26(a). A flow cytometry analysis on Day 7 after the infection regarding the cells which had been infected with Sendai virus capable of expressing NGN2-Puro mRNAs prepared in Example 1 showed that the cells were positive to PSA-NCAM which is a marker for neurons, as shown in Fig. 26(b). Incidentally, the cells were also positive to PSA-NCAM which is a marker for neurons on a flow cytometry analysis on Day 4 after infection.

A flow cytometry analysis on Day 7 after the infection regarding the cells which had been infected with Sendai virus capable of expressing ASCL1-MYT1L-Puro mRNAs and DLX2-Blast mRNAs prepared in Example 11 showed that the cells were positive to PSA-NCAM which is a marker for neurons, as shown in Fig. 26(c). Incidentally, the cells were also positive to PSA-NCAM which is a marker for neurons on a flow cytometry analysis on Day 4 after infection. A flow cytometry analysis on Day 7 after the infection regarding the cells which had been infected with Sendai virus capable of expressing, ASCL1-MYT1L-Puro mRNAs prepared in Example 22 showed that the cells were positive to PSA-NCAM which is a marker for neurons, as shown in Fig. 26(d). Incidentally, the cells were also positive to PSA-NCAM which is a marker for neurons on a flow cytometry analysis on Day 4 after infection.

## Claims

1. A nerve cell production method comprising:
preparing stem cells, and
introducing Sendai virus by infection to the stem cells to induce the stem cells into nerve cells by allowing the Sendai virus to express mRNA which synthesizes an inducing factor in the stem cells.

2. The nerve cell production method according to claim 1, wherein the stem cells are induced pluripotent stem cells.

3. The nerve cell production method according to claim 1, wherein the stem cells are embryonic stem cells.

4. The nerve cell production method according to any one of claims 1 to 3, wherein the inducing factor includes neurogenin (NGN).

5. The nerve cell production method according to any one of claims 1 to 3, wherein the inducing factor includes achaete-scute homolog (ASCL).

6. The nerve cell production method according to any one of claims 1 to 3, wherein the inducing factor includes myelin transcription factor (MYT).

7. The nerve cell production method according to any one of claims 1 to 3, wherein the inducing factor includes distal-less homeobox (DLX).

8. The nerve cell production method according to any one of claims 1 to 7, wherein the nerve cells are of one or more types selected from the group consisting of excitatory neurons, inhibitory neurons, dopamine-producing neurons, locomotor neurons, and cerebral neurons.

9. The nerve cell production method according to any one of claims 1 to 7, wherein the nerve cells are positive for one or more selected from the group consisting of TH, TUJ1, BRN2, NGN, β-III Tubulin, MAP2, PSA-NCAM, vGLUT, SATB2, chAT, HB9, MUNC13, HOMER1, vGAT, and GAD65/67.

10. The nerve cell production method according to any one of claims 1 to 9, wherein the Sendai virus is allowed to express mRNA of a drug resistance gene in the stem cells.

11. The nerve cell production method according to claim 10, wherein the drug resistance gene is one or more selected from the group consisting of puromycin resistance gene, blasticidin resistance gene, hygromycin resistance gene, and neomycin resistance gene.

12. The nerve cell production method according to claim 10 or 11, comprising selecting cells exhibiting drug resistance after infection of the stem cells with Sendai virus.
